(19) Europäisches Patentamt
European Patent Office
Office européen des brevets

(11) EP 1 077 992 B1

(12) EUROPEAN PATENT SPECIFICATION

(45) Date of publication and mention of the grant of the patent:
**10.12.2003 Bulletin 2003/50**

(21) Application number: **99919126.5**

(22) Date of filing: **11.05.1999**

(51) Int Cl.[7]: **C07J 9/00**, C07J 21/00, C07J 41/00, C07J 43/00, A61K 31/575

(86) International application number:
**PCT/DK99/00263**

(87) International publication number:
**WO 99/058549 (18.11.1999 Gazette 1999/46)**

# (54) USE OF MEIOSIS REGULATING COMPOUNDS

VERWENDUNG VON VERBINDUNGEN MIT DER FÄHIGKEIT DIE MEIOSE ZU REGULIEREN

UTILISATION DE COMPOSES REGULATEURS DE LA MEIOSE

(84) Designated Contracting States:
**AT BE CH CY DE DK ES FI FR GB GR IE IT LI LU MC NL PT SE**
Designated Extension States:
**LT RO SI**

(30) Priority:
**13.05.1998 DK 65798**
**14.05.1998 EP 98250166**
**21.05.1998 US 86306 P**
**19.06.1998 DK 81198**
**16.07.1998 US 92983 P**

(43) Date of publication of application:
**28.02.2001 Bulletin 2001/09**

(73) Proprietor: **NOVO NORDISK A/S**
**2880 Bagsvaerd (DK)**

(72) Inventors:
- **FAARUP, Peter**
  **DK-3500 Vaerlose (DK)**
- **GRONVALD, Frederik, Christian**
  **DK-2950 Vedbaek (DK)**
- **BLUME, Thorsten**
  **D-13467 Berlin (DE)**
- **MURRAY, Anthony**
  **DK-2900 Hellerup (DK)**
- **BREINHOLT, Jens**
  **DK-2860 Soborg (DK)**

(56) References cited:
**WO-A1-94/18225** **WO-A1-96/00235**
**WO-A1-98/28323** **WO-A1-98/52965**
**WO-A1-98/55498**

- **NATURE, ANNE GRETE BYSKOV ET AL.,: 'Chemical structure of sterols that activate oocyte meiosis' vol. 374, April 1995, pages 559 - 562, XP002043016**
- **ACTA CHEMICA SCANDINAVICA, MARTIN WENCKENS ET AL.: 'Synthesis of Meiosis-Activating Sterols Containing Fluorine' vol. 52, 1998, pages 503 - 507, XP000857743**
- **LOUIS F. FIESER ET AL.: 'STEROIDS', 1967, REINHOLD PUBLISHING CORPORATION, NEW YORK, pages 400-401**
- **ARCHIVES OF BIOCHEMISTRY AND BIOPHYSICS, W. DAVID NES ET AL.: 'Metabolism of 24(R,S), 25-Epiminolanosterol to 25-Aminolanosterol and Lanosterol by Gibberella fujikuroi' vol. 272, no. 2, 01 August 1989, pages 323 - 331, XP000863784**
- **STN International, File CAPLUS, CAPLUS accession no. 1992:124744, document nr. 116:124744, Ogorodnikova T.E. et al., "The biochemical basis of different nystatin resistance of Saccharomyces cerevisiae and Candida maltosa mutants", XP002921322 & MIKROBIOLOGIYA vol. 60, no. 6, pages 23 - 33, see CASRN 137254-40-1**
- **STN, International, File CAPLUS, CAPLUS accession no. 1991:137944, document no. 114:137944, Ziogas B.N. et al., "Fenpropimorph: a three-site inhibitor of ergosterol biosynthesis in Nectria Haematococca var. cucurbitae, XP002921323 & PESTIC. BIOCHEM. PHYSIOL. vol. 39, no. 1, pages 74 - 83, see CASRN 84964-14-7**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- STN International, File CAPLUS, CAPLUS accession no. 1991:639433, document no. 115:239433, Jirovetz L. et al., "Investigation of the composition and effects of grass flowers", XP002921324 & SEIFEN, OELE, FETTE, WACHSE, vol. 117, no. 7, 1991, pages 252 - 256 (German), see CASRN 1137331-75-0
- STN International, File CAPLUS, CAPLUS accession no. 1993:472880, document no. 119:72880, DeKeczer Steve et al., "Synthesis of (14.alpha.-methyl-3h)-24,25-dihydrolanosterol", XP002921325 & J. LABELLED COMPD. RADIOPHARM. vol. 33, no. 3, 1993, pages 219-231 (Eng.) see CASRN 53296-71-2
- STN International, File CAPLUS, CAPLUS accession no. 1996:386686, document no. 125:81848, Akihisa Toshihiro et al., "Eight novel sterols from the roots of Bryonia dioica Jacy.", XP002921326 & CHEM. PHARM. BULL. vol. 44, no. 6, 1996, pages 1202-1207, (Eng.) see CASRN 178275-56-4
- STN International, File CAPLUS, CAPLUS accession no. 1997:73595, document no. 126:183757, Raldugin V. A. et al., "Triterpenoids from Abies species.19. (22z)-3.alpha.-Hydroxy-17,14-friedolanosta-7,14,22,24-tetraene-26,23-olide, a new lactone from needles of Siberian fir.", XP002921327 & IZV. AKAD. NAUK, SER. KIM. vol. 11, 1996, pages 2792 - 2794 (Russian), see CASRN 139483-01-5
- Herz et al. Steroids (1976) 27(1), 133-6

## Description

The present invention relates to certain pharmacologically active compounds, to pharmaceutical compositions containing certain compounds as active substance and to their use as medicaments. More particularly, it has been found that the derivatives described herein can be used for regulating the meiosis.

BACKGROUND OF THIS INVENTION

Meiosis is the unique and ultimate event of germ cells on which sexual reproduction is based. Meiosis comprises two meiotic divisions. During the first division, exchange between maternal and paternal genes take place before the pairs of chromosomes are separated into the two daughter cells. These contain only half the number (1n) of chromosomes and 2c DNA. The second meiotic division proceeds without a DNA synthesis. This division, therefore, results in the formation of the haploid germ cells with only 1c DNA.

The meiotic events are similar in the male and female germ cells, but the time schedule and the differentiation processes which lead to ova and to spermatozoa differ profoundly. All female germ cells enter the prophase of the first meiotic division early in life, often before birth, but all are arrested as oocytes later in the prophase (dictyate state) until ovulation after puberty. Thus, from early life the female has a stock of oocytes which is drawn upon until the stock is exhausted. Meiosis in females is not completed until after fertilisation, and results in only one ovum and two abortive polar bodies per germ cell. In contrast, only some of the male germ cells enter meiosis from puberty and leave a stem population of germ cells throughout life. Once initiated, meiosis in the male cell proceeds without significant delay and produces 4 spermatozoa.

Only little is known about the mechanisms which control the initiation of meiosis in the male and in the female. In the oocyte, new studies indicate that follicular purines, hypoxanthine or adenosine, could be responsible for meiotic arrest (Downs, S.M. *et al. Dev.Biol.* **82** (1985) 454-458; Eppig, J.J. *et al. Dev. Biol.* **119** (1986) 313-321; and Downs, S.M. *Mol.Reprod.Dev*. **35** (1993), 82-94). The presence of a diffusible meiosis regulating substance was first described by Byskov *et al*. in a culture system of fetal mouse gonads (Byskov, A.G. *et al*. in *Dev.Biol.* **52** (1976), 193-200). A meiosis activating substance (MAS) was secreted by the fetal mouse ovary in which meiosis was ongoing, and a meiosis preventing substance (MPS) was released from the morphologically differentiated testis with resting, non-meiotic germ cells. It was suggested that the relative concentrations of MAS and MPS regulated the beginning, arrest and resumption of meiosis in the male and in the female germ cells (Byskov, A.G. *et al*. in *The Physiology of Reproduction* (eds. Knobil, E. and Neill, J.D., Raven Press, New York (1994)). Clearly, if meiosis can be regulated, reproduction can be controlled. A recent article (Byskov, A.G. *et al*. in *Nature* **374** (1995), 559-562) describes the isolation from bull testes and from human follicular fluid of certain sterols that activate oocyte meiosis. Unfortunately, these sterols are rather labile and utilisation of the interesting finding would thus be greatly facilitated if more stable meiosis activating compounds were available.

In *Aust.J.Chem.* **35** (1982), 629-640, Horn et *al*. deals with compounds possibly having biological activity (insect moulting hormones). Examples of compounds specifically mentioned therein are 5-cyano-5β-cholest-7-en-3-one; 5-cyano-5β-cholest-7-en-3β-ol; 5-methyl-5β-cholest-7-en-3-one; 5-methyl-5β-cholest-7-en-3α-ol; and 5-methyl-5β-cholest-7-en-3β-ol.

In *Bull.Soc.Chim.Fr.* (1971), 2037-2047, Levisalles *et al*., cholesta-4,8(14)-dien-3-one is described as an intermediate.

In *Just.Lieb.Ann.Chem*. **542** (1939), 218-224, Windaus *et al*. mentions cholesta-4,7-dien-3-one; cholesta-4,7-dien-3α-ol; and cholesta-4,7-dien-3β-ol as intermediates.

In *Pharm.Bull.* **1** (1953), 224-227, Arima mentions cholesta-4,8-dien-3-one as intermediate.

In *Lipids* **13** (1978), 704 *et seq*., Kandutsch *et al*. describes some cholestane derivatives which may be potent inhibitors of sterol synthesis. Compounds specifically mentioned therein are, in Fig. 1, 3β,7α-dihydroxycholest-5-ene; 3β,7β-dihydroxycholest-5-ene; 3β-hydroxycholest-5-en-7-one; 3β-hydroxycholest-7-one; 7α-hydroxycholest-4-en-3-one; in Fig. 2 (compounds 1-5), cholest-3,6-dione; 3β-hydroxycholest-6-one; 3β,6β-dihydroxycholestane; cholest-4-en-3,6-dione; 3β,5α,6β-trihydroxycholestane; in Fig. 3, 3β,5α-dihydroxycholestane; 3β,4β-dihydroxycholest-5-ene; and, in Fig. 4 (compounds 1, 3, 4 and 5), cholest-2-en-6-one; cholest-4,6-dien-3-one; cholest-4,7-dien-3-one; cholest-3,5-dien-7-one.

Danish patent application with publication number 130,992, deals with compounds possibly having progestomimetic properties. Examples of compounds specifically mentioned therein are 19-nor-21-methylpregna-4,9-dien-17α-hydroxy-3,20-dione; 19-nor-21-methyl-pregna-4,9-dien-17α-acetoxy-3,20-dione; 19-nor-21,21-dimethylpregna-4,9-diene-17α-hydroxy-3,20-dione; and 17α-21,21-dimethyl-19-nor-pregna-4,9-dien-3,20-dione.

In the Danish patent application with publication number 136,909, 3α-acetoxy-24-nor-cholan-23-one; 3α-hydroxy-26,27-di-nor-23-trans-5β-cholest-23-en-25-carboxylic acid methyl ester; 3α-hydroxy-26,27-di-nor-5β-cholesta-25-carboxylic acid methyl ester; 3-keto-26,27-di-nor-5β-cholesta-25-carboxylic acid methyl ester, 3-keto-4-bromo-26,27-di-nor-5β-cholesta-25-carboxylic acid methyl ester; 3-keto-26,27-di-nor-cholest-4-en-25-carboxylic acid methyl ester; 3β-acetoxy-26,27-di-nor-cholesta-3,5-dien-25-carboxylic acid methyl ester; 3β-hydroxy-26,27-di-nor-cholest-5-en-25-carboxylic acid methyl ester; 3-keto-26,27-di-norcholest-4, 6-dien-25-carboxylic acid methyl ester; 3β-hydroxy-26,27-di-nor-cholesta-3,5,7-trien-25-carboxylic acid methyl ester; and 3β-hydroxy-26,27-di-nor-cholesta-5,7-dien-25-carboxylic acid methyl ester are mentioned as intermediates.

Danish patent application with publication number 146,390, deals with compounds possibly having pharmacological properties, e.g. an inhibiting action on the production of serum cholesterol. Examples of compounds specifically mentioned therein are 3β,22-diacetoxycholesta-5-en-25-ol; 3β,22-diacetoxy-25-fluorocholesta-5-ene; 22-hydroxycholesta-5-en-25-fluoro-3β-hemisuccinate; 3β,22-diacetoxy-25-dichlorocholesta-5-ene; 3β,22-dihydroxy-25-chlorocholesta-5-ene; 22-hydroxy-25-chlorocholesta-5-en-3β-hemisuccinate; 3β,22-dihydroxy-25-bromocholesta-5-ene; and 3β,22-dihydroxy-25-fluorocholesta-5-ene.

In the Danish patent applications with publication numbers 156,726 and 156,644, cholenic acid; 3β-acetoxycholesta-5-en-25-des-dimethyl-24-one; 3,24-diacetoxycholesta-25-des-dimethyl-5, 23-diene; 3β-acetoxycholesta-25-des-methyl-5-en-24-difluoro-25-one; and 3β-acetoxy-24-difluorocholesta-5,7-dien-25-ol are mentioned as intermediates.

In the Danish patent application with publication number 158,790, 3β-hydroxycholest-5-en-24-one; 3β-acetoxycholest-5-en-24-one; 5β-cholest-24-one; 5β-cholestan-24α-homo-24-one; 3α,6α-dihydroxy-5β-cholest-24-one; 3α,6α-diacetoxy-5β-cholest-24-one; 3α,6α-diacetoxy-5β-cholest-24α-homo-24-one; 3α,6α-dihydroxy-5β-cholest-24α,24β-bis-homo-24-one; 3α-hydroxy-5β-cholest-24-one; 3α-acetoxy 5β-cholesta-24-one; 3α-benzoyloxy-5β-cholesta -24-one; 3α-ethyloxycarbonyloxy-5β-cholesta- 24-one; 3α-hydroxy-5β-cholestan-24α-homo-24-one; 3α-hydroxy-24α,24β-bis-homo-5β-cholestane; 3β-hydroxycholesta-5,7-dien-24-one; 3β-acetoxycholesta-5,7-dien-24-one; 1α,3β-dihydroxycholesta-5,7-diene-24-one; and 1α,3β-diacetoxycholesta-5,7-diene-24-one are mentioned as intermediates.

Danish patent application with publication number 159,456, deals with compounds possibly having utility in the treatment of gall diskinese. Examples of compounds specifically mentioned therein are chenodeoxycholic acid; ursodeoxycholic acid; trimebutyn salt of chenodeoxycholic acid; and trimebutyn salt of ursodeoxycholic acid.

In the Danish patent application with publication number 162,648, 3β,25-dihydroxycholest-5-en-24-one; 3β-acetoxycholest-5-en-24-one; 3β-acetoxy-25-hydroxycholest-5-en-24-one; 3β,25-dihydroxycholest-5-en-24-one; 3β-hydroxycholest-5-en-24-one; 3β-hydroxy-25-hydroperoxycholest-5-en-24-one; 3β,24,25-trihydroxycholest-5-ene; 1α,3β-dihydroxycholest-5-en-24-one; 1α,3β,25-trihydroxycholest-5-en-24-one; 1α,3β,24,25-tetrahydroxycholest-5-en-24-one are mentioned as intermediates.

In the Danish patent application with publication number 165,410, 3α-acetoxy-7α-bromocholest-5-ene; 3α-acetoxycholesta-5,7-diene; and 3α-acetoxy-25-hydroxycholesta-5,7-diene are mentioned as intermediates.

In the Danish patent application with publication number 165,695, 3β,25-dihydroxy-26,27-hexafluorocholest-5-ene; and 1α,3β,25-trihydroxy-26,27-hexafluorocholest-5-ene are mentioned as intermediates for the preparation of vitamin D analogues.

Danish patent application with publication number 167,220 deals with compounds possibly having utility for the treatment of liver disorders. An examples of a compound specifically mentioned therein is 3α,7α,12α,24R,26,27-hexahydroxycholestane.

In U.S. patent No. 4,425,274, the compounds 3α-hydroxy-7-cholanic acid; 3α,7α-dihydroxycholanic acid; 3α,7β-dihydroxycholanic acid; and lithium 3α,7β-dihydroxycholanate are described as intgermediates.

In the Norwegian patent application with publication number 144,264, cholesta-1,4,-6-trien-3-one; cholest-5-en-1α,3β-diol; 1α-hydroxycholesta-4,6-diene-3-one; 1α,3β-dihydroxycholest-5-ene; 25-hydroxycholesta-1,4,6-triene-3-one; and 1α,3β-25-trihydroxycholest-5-ene are mentioned as intermediates for the preparation of 1α-hydroxy steroids of the cholestane serie.

Norwegian patent application with publication number 158,423, deals with compounds possibly having utility in the treatment of biliary dyskinesis. An example of a compound specifically mentioned therein is 3α,7β-dihydroxydeoxycholic acid.

In the Norwegian patent application with publication number 162,562, 3α,7α-dihydroxydeoxycholic acid; 7-ketodeoxycholic acid; 3α,7β-dihydroxydeoxycholic acid; cholic acid; 7-ketocholic acid; 3α,7β,12α-cholic acid; and 12-ketocholic acid are mentioned as being intermediates in the preparation of ursodeoxycholanic acid.

In the Norwegian patent application with publication number 162,665, cholic acid; 3α-hydroxy-7-ketocholic acid; 3α,7α-diacetoxycholic acid; 3α,7α-diacetoxy-12-ketocholic acid; 3α,7α-dihydroxydeoxycholic acid; 7-ketodeoxycholic acid; 3α,7β-dihydroxydeoxycholic acid; and 3α,7β-dihydroxy-12-ketocholic acid are mentioned as being intermediates in the preparation of ursodeoxycholanic acid.

In the Norwegian patent application with publication number 303,450, cholic acid; cholic acid methyl ester; 3-acetylcholic acid methyl ester; 3-(2-propenyl)cholic acid methyl ester; 3-(3-hydroxypropyl)cholic acid-3-methyl ester; desoxycholic acid; 12-keto-desoxycholic acid; 3β-acetyloxy-12-keto-desoxycholic acid; 3β-(hydroxyethyloxy)cholic acid methyl ester; 3β-(hydroxypropyloxy)cholic acid methyl ester; 3β-(hydroxybutyloxy)cholic acid methyl ester; 3β-(hydroxypentyloxy)cholic acid methyl ester; 3β-(hydroxyhexyloxy)cholic acid methyl ester; 3β-(hydroxydecanoyloxy)cholic acid methyl ester; 3β-(2-hydroxyethyloxyethyloxy)cholic acid methyl ester; 3β-(2-hydroxypropyloxy)cholic acid methyl ester; 3β-(hydroxyethyloxy)desoxycholic acid methyl ester; 3β-(hydroxypropyloxy)desoxycholic acid methyl ester; 3β-(hydroxypentyloxy)desoxycholic acid methyl ester; 3β-(hydroxydecyloxy)desoxycholic acid methyl ester; 3β-(2-hydroxyethyloxy)chenodesoxycholic acid methyl ester; 3β-(3-hydroxypropyloxy)-chenodesoxycholic acid methyl ester; 3β-(5-hydroxypentyloxy)chenodesoxycholic acid methyl ester; 3β-(10-hydroxydecyloxy)chenodesoxycholic acid methyl ester; 3β-(2-hydroxyethyloxy)litocholic acid methyl ester; 3β-(3-hydroxypropyloxy)litocholic acid methyl ester; 3β-(5-hydroxypenthyloxy)lithocholic acid methyl ester; 3β-(10-hydroxydecayloxy)lithocholic acid methyl ester; 3β-(benzyloxyethyloxy)cholic acid methyl ester; 3β-(benzyloxyethyloxy)cholic acid *tert*.butyl ester; 3β-(2-hydroxyethyloxy)cholic acid *tert*. butyl ester; 3β-(2-hydroxyethyloxy)-7α,12α-diacetyloxycholic acid methyl ester; and 3β-(propionyloxy)-7α,12α-diacetyloxy-24-carboxylic acid methyl ester are mentioned as intermediates.

In the Swedish patent application with publication number 385,905, chenodeoxycholic acid is mentioned to have utility for the treatment of cholelithiasis and cholic acid is mentioned as an intermediate for the preparation thereof.

Swedish patent application with publication number 402,462 mentions sitosterol which may have medical application, e.g. for the prevention or reduction of absorption of cholesterol in the small intestine, and campesterol is mentioned to lower the effect of sitosterol.

In the Swedish patent application with publication number 413,247, 3α-hydroxycholestane and 3β-hydroxycholestane are mentioned to have antiinflammatoric properties and slightly side effects.

Swedish patent application with publication number 430,508 deals with compounds possibly having pharmacological properties, i.e. inhibition of HMG-CoA reductase and inhibition of the formation of serum cholesterol. Examples of compounds specifically mentioned therein are 25-fluorocholest-5-en-3β,22-diol; 25-chlorocholest-5-en-3β,22-diol; 22-hydroxy-25-fluorocholest-5-en-3β-hemisuccinate; 22-hydroxy-25-chlorocholest-5-en-3β-hemisuccinate; and cholesta-5-en-3β,22,25-triol.

Compounds being known to stimulate the meiosis and being different from the compounds claimed in the present patent application are described in International patent applications having Nos. WO 96/00235, 96/27658 and 97/00884 (Novo Nordisk A/S) and 98/55498. In International patent application having No. WO 98/52965, filed on 11 May 1998 and published on 26 November 1998, it is stated that certain 20-aralkyl-5α-pregnan derivatives can be used in the preparation of a medicament for the control of fertility and some of the specific compounds mentioned therein are (3β,5α,20R)-4,4,20-trimethyl-21-phenylpregna-8,14-dien-3-ol (example 1); (3β,5α,20R)-4,4,20-trimethyl-21-(3-methylphenyl)pregna-8,14-dien-3-ol (example 2A); and (3β,5α,20R)-4,4-dimethyl-23-phenyl-24-norchola-8,14-dien-3-ol (example 7A).

The compounds described herein have advantages compared with the known compounds.

## SUMMARY OF THIS INVENTION

A main purpose of this invention is to furnish compounds which can be used to regulate meiosis.

One purpose of the present invention is to provide compounds and methods useful for relieving infertility in females and males, particularly in mammals, more particularly in humans.

In a further object, the present invention concerns the use of the compounds of the general formula I (stated in the claims, below) for relieving infertility in females and males, particularly in mammals, more particularly in humans.

In a further object of the present invention the compounds of the general formula I are useful as contraceptives in females and males, particularly in mammals, more particularly in humans.

In a further preferred embodiment, this invention relates to compounds of the general formula I (stated in the claims below) or esters or salts thereof in the manufacture of a medicament for use in the regulation of meiosis.

In a further preferred aspect, the present invention relates to the use of a compound of formula I or an ester or salt thereof as a medicament, in particular as a medicament for use in the regulation of meiosis. The compound may be used neat or in the form of a liquid or solid composition containing auxiliary ingredients conventionally used in the art.

In the present context, the expression "regulating the meiosis" is used to indicate that certain of the compounds of formula I can be used for stimulating the meiosis *in vitro, in vivo,* or *ex vivo*. Thus, the compounds of formula I which may be agonists of a naturally occurring meiosis activating substance, can be used in the treatment of infertility which is due to insufficient stimulation of meiosis in females and in males. Other compounds of formula I, which may be antagonists of a naturally occurring meiosis activating substance, can be used for regulating the meiosis, preferably *in vivo*, in a way which makes them suited as contraceptives. In this case the "regulation" means partial or total inhibition.

In a still further preferred aspect, the present invention relates to the use of a compound of formula I or an ester or salt thereof in the regulation of the meiosis of an oocyte, in particular a mammalian oocyte, more particularly a human oocyte.

In a still further preferred aspect, the present invention relates to the use of a compound of formula I or an ester or salt thereof in the stimulation of the meiosis of an oocyte, in particular a mammalian oocyte, more particularly a

human oocyte.

In a still further preferred aspect, the present invention relates to the use of a compound of formula I or an ester or salt thereof in the inhibition of the meiosis of an oocyte, in particular a mammalian oocyte, more particularly a human oocyte.

In a still further preferred aspect, the present invention relates to the use of a compound of formula I or an ester or salt thereof in the regulation of the meiosis of a male germ cell, in particular a mammalian male germ cell, more particularly a human male germ cell.

In a still further preferred aspect, the present invention relates to the use of a compound of formula I or an ester or salt thereof in the stimulation of the meiosis of a male germ cell, in particular a mammalian male germ cell, more particularly a human male germ cell.

In a still further preferred aspect, the present invention relates to the use of a compound of formula I or an ester or salt thereof in the inhibition of the meiosis of a male germ cell, in particular a mammalian male germ cell, more particularly a human male germ cell.

In a yet still further preferred aspect, the present invention relates to a method of regulating the meiosis in a mammalian germ cell which method comprises administering an effective amount of a compound of formula I or an ester or salt thereof to a germ cell in need of such a treatment.

In a still further aspect, the present invention relates to a method of regulating the meiosis in a mammalian germ cell wherein a compound of formula I or an ester or salt thereof is administered to the germ cell by administering the compound to a mammal hosting said cell.

In a still further aspect, the present invention relates to a method wherein the germ cell the meiosis of which is to be regulated by means of a compound of formula I or an ester or salt thereof is an oocyte.

In a still further aspect, the present invention relates to a method of regulating the meiosis in an oocyte wherein a compound of formula I or an ester or salt thereof is administered to the oocyte *ex vivo*.

In a still further aspect, the present invention relates to a method of regulating the meiosis of a male germ cell by administering a compound of formula I or an ester or salt thereof to the cell.

In a still further aspect, the present invention relates to a method whereby mature male germ cells are produced by administering *in vivo* or *in vitro* a compound of formula I or an ester or salt thereof to testicular tissue containing immature cells.

The compounds of formula I are useful for regulating the meiosis in oocytes and in male germ cells.

DETAILED DESCRIPTION OF THIS INVENTION

It has, surprisingly, been found that compounds having a side chain ($R^{22}$) which is different from the cholesterol and lanosterol side chains or compounds having certain specifically elected substituents in the ring system, have superior properties.

Preferred compounds of formula I are such having a double bond.

Other preferred compounds of formula I are such wherein $R^3$ is hydrogen.

Other preferred compounds of formula I are such wherein $R^3$ is perfluoro($C_1$-$C_6$ alkyl).

Other preferred compounds of formula I are such wherein $R^4$ and $R'^4$ are both hydrogen.

Other preferred compounds of formula I are such wherein one of $R^4$ and $R'^4$ is hydrogen while the other is methyl.

Other preferred compounds of formula I are such wherein $R^4$ and $R'^4$ are both methyl.

Other preferred compounds of formula I are such wherein $R^4$ is branched or unbranched $C_1$-$C_6$ alkyl.

Other preferred compounds of formula I are such wherein $R^5$ is hydrogen.

Other preferred compounds of formula I are such wherein $R^5$, together with $R^4$, is an additional bond.

Other preferred compounds of formula I are such wherein $R^7$, together with $R^8$, designates an additional bond between the carbon atoms at which $R^7$ and $R^8$ are placed.

Other preferred compounds of formula I are such wherein $R^8$, together with $R^9$, designates an additional bond between the carbon atoms at which $R^8$ and $R^9$ are placed.

Other preferred compounds of formula I are such wherein $R^8$ is hydrogen.

Other preferred compounds of formula I are such wherein $R^9$ is hydrogen.

Other preferred compounds of formula I are such wherein $R^{14}$ is hydrogen.

Other preferred compounds of formula I are such wherein $R^{15}$ is hydrogen.

Other preferred compounds of formula I are such wherein $R^{15}$, together with $R^{14}$, designates an additional bond between the carbon atoms at which $R^{15}$ and $R^{14}$ are placed.

Other preferred compounds of formula I are such wherein $R^{16}$ is hydrogen.

Other preferred compounds of formula I are such wherein $R^{16}$ together with $R^{17}$, designates an additional bond between the carbon atoms at which $R^{16}$ and $R^{17}$ are placed.

Other preferred compounds of formula I are such wherein $R^{17}$ is hydrogen.

Other preferred compounds of formula I are such wherein $R^{17}$ is in the $\alpha$ position.

Other preferred compounds of formula I are such wherein the side chain in the 17 position (i.e. $-C(R^{20})(R'^{20})-CH(R^{22})(R'^{22})$) is in the $\beta$ position.

Other preferred compounds of formula I are such wherein $R^1$ is hydrogen; $R^2$ is hydrogen; $R^3$ is hydrogen; $R^4$ and $R^{4'}$, which are different or identical, are selected from the group comprising hydrogen and branched or unbranched $C_1$-$C_6$ alkyl, or $R^4$ and $R^{4'}$ together designate methylene; $R^5$ is hydrogen, or $R^5$ designates, together with $R^6$, an additional bond between the carbon atoms at which $R^5$ and $R^6$ are placed; $R^6$ is hydrogen; $R^7$ is hydrogen, or $R^7$ designates, together with $R^8$, an additional bond between the carbon atoms at which $R^7$ and $R^8$ are placed; $R^8$ is hydrogen, or $R^8$ designates, together with $R^7$, $R^9$ or $R^{14}$, an additional bond between the carbon atoms at which $R^8$ and $R^7$, $R^9$ or $R^{14}$ are placed; $R^9$ is hydrogen, or $R^9$ designates, together with $R^8$ or $R^{11}$, an additional bond between the carbon atoms at which $R^9$ and $R^8$ or $R^{11}$ are placed; $R^{11}$ is hydrogen; $R^{12}$ is hydrogen; $R^{14}$ is hydrogen, or $R^{14}$ designates, together with $R^{15}$, an additional bond between the carbon atoms at which $R^{14}$ and $R^{15}$ are placed; $R^{15}$ is hydrogen; $R^{16}$ is hydrogen, or $R^{16}$ designates, together with $R^{17}$, an additional bond between the carbon atoms at which $R^{16}$ and $R^{17}$ are placed; $R^{17}$ is hydrogen; $R^{20}$ is $C_1$-$C_4$ alkyl; $R^{20'}$ is hydrogen; $R^{22'}$ is hydrogen; and $R^{22}$ is cyclohexyl; and esters thereof.

Other preferred compounds of formula I are such wherein $R^5$ is a $C_1$-$C_3$-alkyl group, preferably a methyl group, a cyano group, a hydroxymethyl group or together with $R^4$ a methano bridge or together with $R^4$ an additional bond.

It is to be understood that the above preferred substituents can be combined in any way with each other.

Examples of interesting and preferred compounds of the general formula I are mentioned in claim 2, below.

Preferred compounds of formula I are such which when tested by the method described below for agonistic properties (example 25) shows a relative activity of at least 50, preferably at least 80, or when tested by the method described below for antagonistic properties (example 26) shows a $IC_{50}$ value below 10, preferably below 2.

Examples of other preferred compounds are such not being active at the oestrogen receptor, and preferably compounds not being active at other hormone receptors.

Further preferred embodiments are mentioned in the appended claims.

As used in the present description and claims, a lower alkyl group - when used alone or in combinations - may be a straight or branched alkyl group. Said alkyl group contains not more than 6 carbon atoms. Examples of preferred alkyl groups are methyl, ethyl, propyl, isopropyl, butyl, *tert*-butyl, pentyl and hexyl, more preferred methyl, ethyl, propyl, isopropyl, butyl and *tert*-butyl, still more preferred methyl and ethyl. In a preferred embodiment of this invention, the alkyl group contains not more than 4 carbon atoms, preferably not more than 3 carbon atoms.

Salts of compounds of formula I are preferably pharmaceutically acceptable salts, especially acid-addition salts, including salts of organic acids and mineral acids. Examples of such salts include salts of organic acids such as formic acid, fumaric acid, acetic acid, propionic acid, glycolic acid, lactic acid, pyruvic acid, oxalic acid, succinic acid, malic acid, tartaric acid, citric acid, benzoic acid, salicylic acid and the like. Suitable inorganic acid-addition salts include salts of hydrochloric, hydrobromic, sulphuric and phosphoric acids and the like. Further examples of pharmaceutically acceptable inorganic or organic acid addition salts include the pharmaceutically acceptable salts listed in *Journal of Pharmaceutical Science*, **66** (1977), 2 *et seq*.

Esters of compounds of formula I are formally derived by esterification of one or more hydroxylic groups of a compound of formula I, respectively, with an acid which can for example be selected from the group of acids comprising succinic acid and other aliphatic dicarboxylic acids, nicotinic acid, isonicotinic acid, ethylcarbonic acid, phosphoric acid, sulphonic acid, sulphamic acid, benzoic acid, acetic acid, propionic acid and other aliphatic monocarboxylic acids.

The compounds of formula I have a number of chiral centres in the molecule and thus exist in several isomeric forms. All these isomeric forms and mixtures thereof are within the scope of the invention.

The compounds of the general formula I can be prepared analogously with the preparation of known compounds. Hence, synthesis of the compounds of formula I can follow the well established synthetic pathways described in the comprehensive sterol and steroid literature. The following books can be used as the key source in the synthesis: L.F. Fieser & M. Fieser: Steroids: Reinhold Publishing Corporation, NY 1959; Rood's Chemistry of Carbon Compounds (editor: S. Coffrey): Elsevier Publishing Company, 1971; J. Fried and J.A. Edwards: Organic Reactions in Steroid Chemistry, Vol. I and II, Van Nostrand Reinhold Company, New York, 1972; and especially Dictionary of Steriods (editors: R.A. Hill; D.N. Kirk; H.L.J. Makin and G.M. Murphy): Chapmann & Hall. The last one contains an extensive list of citations to the original papers covering the period up to 1990. All these books including the last mentioned citations are incorporated by reference. In addition, information in all the above publications (including patent specifications) dealing with preparation of compounds similar with compounds of formula I is incorporated by reference.

Particularly, the compounds of the present invention may be synthesised according to the following general procedures:

Cholesta-5,8-dien-3-ol **1**, which is synthesised as described in the literature [*J.Lip.Res*. **37,** 1529, (1996)], can be oxidised in an Oppenauer reaction to give cholesta-4,8-dien-3-one **2** (scheme 1). In this reaction, the sterol is

treated with a ketone like acetone, quinone or cyclohexanone in the presence of aluminum isopropoxide or aluminum *tert*-butoxide [e.g. *J. Chem.Soc.Perkin I* 2667 (1994)]. The sterol can also be oxidised with pyridinium dichromate [*vide Synth.Commun.* **20** (1990), 1167]. The same oxidation reaction can be carried out with cholesta-5,8(14)-dien-3β-ol, which is also synthesised as described in the literature [*J.Lip. Res.* **37** (1996), 1529,] to give cholesta-5,8(14)-dien-3-one. For this ketone, a laborious synthesis is described in the literature [*Bull. Soc. Chim. Fr.* 2037, (1971)]. Cholesta-4,7-dien-3-one is synthesised according to literature procedures [*Liebigs Ann.Chem.* **542** (1939), 218,] (in scheme 1, the series with the $\Delta^8$-double bond are shown as an example, analogous reactions have to be performed in the $\Delta^7$- and $\Delta^{8(14)}$-series).

In the following, only the syntheses in the $\Delta^8$-series are described. The derivatives in the $\Delta^7$ and $\Delta^{8(14)}$-series can be synthesised by a skilled artisan from the corresponding starting materials in the same way.

**scheme 1:**

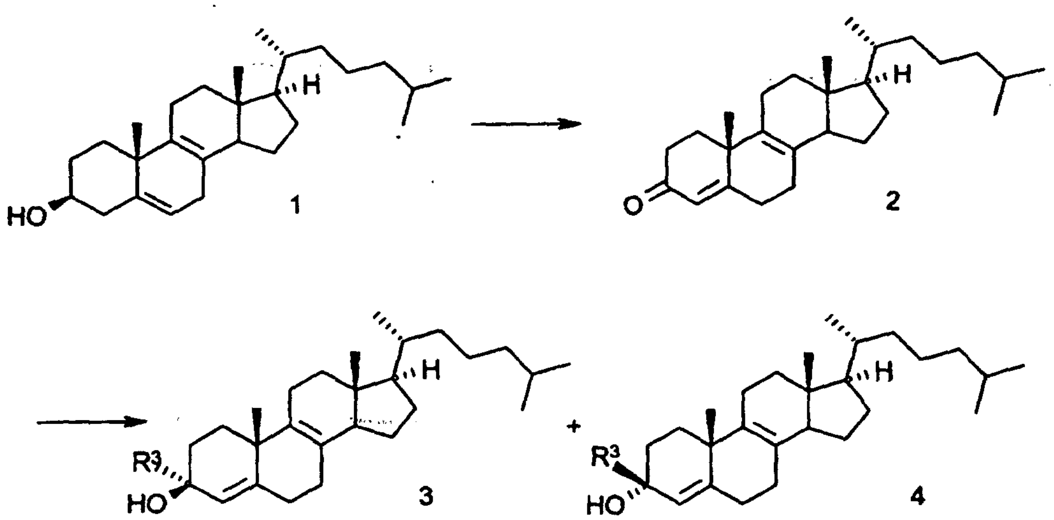

1 2 3 4

Enone **2** can be treated with different $C_1$-$C_6$-Grignard reagents to give two diastereomeric alcohols **3** and **4** (with $R^3$ = $C_1$-$C_6$-alkyl), which are easily separated by column chromatography [e.g. *J.Med.Chem.* **40** (1997), 61].

Cholesta-4,8-dien-3-one **2** can be reduced according to well known literature procedures. Lithium aluminum hydride, sodium borohydride and diisobutylaluminum hydride are especially useful [e.g. *Liebigs Ann. Chem.* **542** (1939), 218]. Two diastereomeric alcohols of the formulae **3** and **4** (with $R^3$ = hydrogen) can be obtained and readily separated by column chromatography.

For the introduction of perfluoroalkyl substituents in position 3, cholesta-4,8-dien-3-one **2** can be treated with perfluoroalkyltrialkylsilanes in the presence of fluoride sources like tetrabutyl-ammonium fluoride or caesium flouride. The trifluoromethyl group is preferentially introduced with reagents like trimethylsilyltrifluoromethane or triethylsilyltrifluoromethane [*J. Org.Chem.* **56** (1991), 984; *J.Org.Chem.* **54** (1989), 2873].

Again two diastereomeric alcohols of the formulae **3** and **4** (with $R^3$ = perfluoroalkyl, preferentially: trifluoromethyl) can be obtained and readily separated by column chromatography.

The cyanoketones **5** and **6** are available starting from cholesta-4,8-dien-3-one **2** via a conjugate addition of cyanide (scheme 2). Different reagents like diethylaluminum cyanide [*J.Org.Chem.* **59** (1994), 2766] and some alkali and earth alkali metal cyanides [*Tetrahedron Lett.* **28** (1987), 4189; *Can.J.Chem.* **59** (1981),1641] can be used in this reaction.

scheme 2:

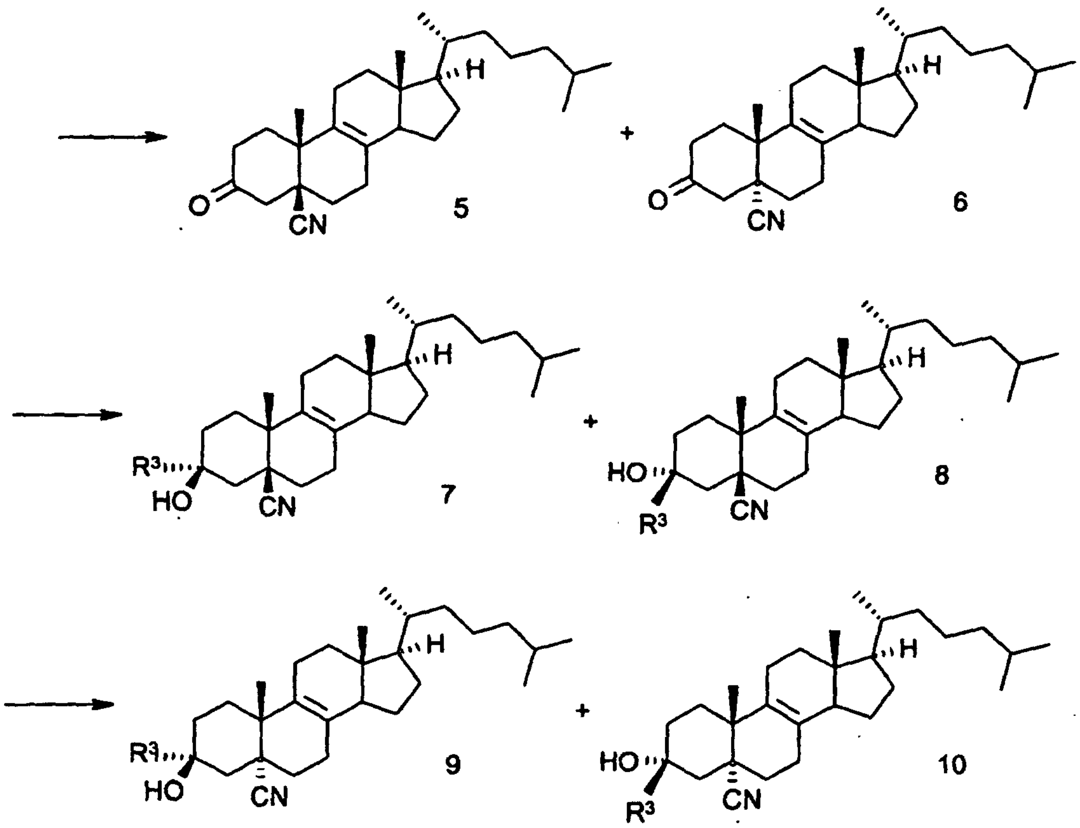

5 + 6

7 + 8

9 + 10

The readily separated cyanoketones **5** and **6** can be reduced according to well known literature procedures. Lithium aluminum hydride, sodium borohydride and diisobutyl-aluminum hydride are used preferentially [e.g. *Aust.J. Chem.* **35** (1982), 629]. In the reduction reactions, two diastereomeric alcohols **7** and **8** ($R^3$ = H), respectively, **9** and **10** ($R^3$ = H) are obtained.

The cyanoketones of the formulae **5** and **6** can also be treated with Grignard reagents [e.g. *Chem.Pharm.Bull.* **9** (1961), 854] to give two diastereomeric tertiary alcohols **7** and **8** or **9** and **10** (with $R^3$ = $C_1$-$C_6$-alkyl), respectively.

The hydroxymethyl derivatives of the formulae **12** and **14** can be synthesised in two step sequences from the cyanoalcohols **8** and **9,** respectively (scheme 3). First the cyano group can be reduced with an electrophilic reducing agent like diisobutylaluminum hydride to give the corresponding imines, which are hydrolysed in situ to the carbaldehydes **11** and **13.** In the second step, the carbaldehydes can be further reduced with well known reducing agents like lithium aluminum hydride, sodium borohydride or diisobutylaluminum hydride to the desired hydroxymethyl derivatives [e.g. *J.Med.Chem.* **39** (1996), 5092].

scheme 3:

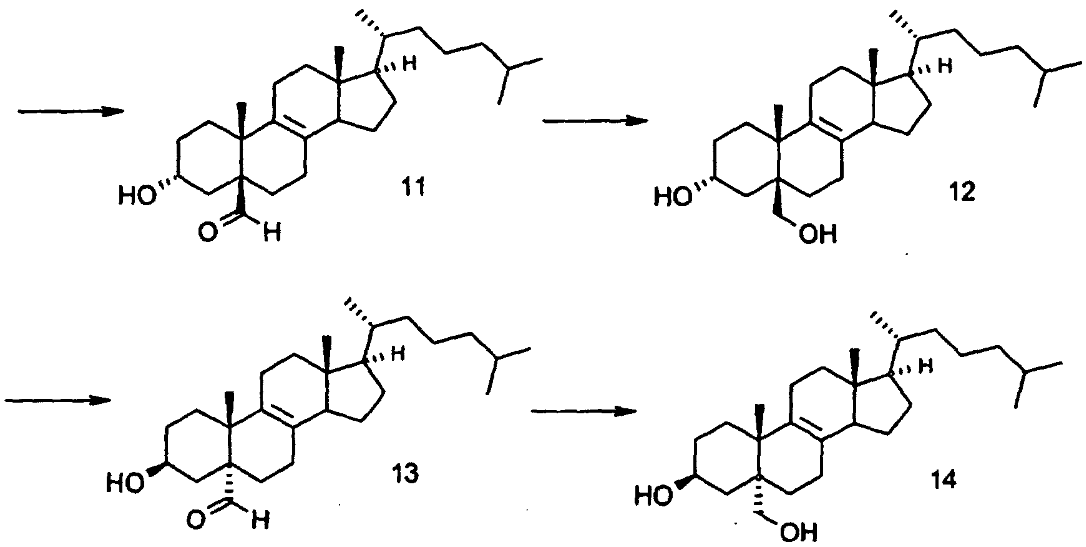

11

12

13

14

The methano derivatives of the formulae **15** and **16** can be synthesised form the allylic alcohols **3** and **4,** respectively (scheme 4). Different variations of the Simmons-Smith reaction can be employed. As reagents, diiodomethane in the presence of zinc/copper-coupte [e.g. *J.Org.Chem.* **31** (1966), 3869; *J.Med.Chem.* **39** (1996), 4218] as well as chloroiodomethane in the presence of a diethylzinc solution [*J.Am.Chem.Soc.* **108** (1986), 6343] can be used in this cyclopropanation reaction.

scheme 4:

3

4

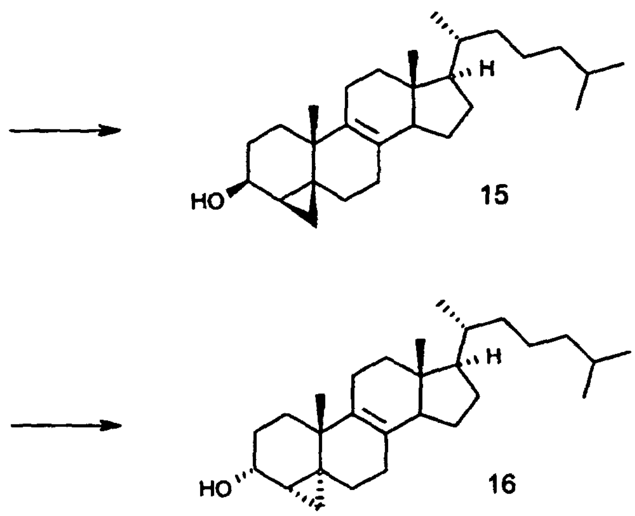

15

16

5β-Methylcholest-8-en-3-one **17** can be synthesised from cholesta-4,8-dien-3-one **2** via a conjugate addition reaction (scheme 5). The methyl group is introduced either with lithium dimethylcuprate [e.g. *Aust.J. Chem.* **35** (1982), 629] or with methyl-Grignard compounds or trimethylaluminum in the presence of nickel catalysts [e.g. *Tetrahedron Lett.* **35** (1994), 6075; *Synthesis* **3** (1995), 317].

scheme 5:

2

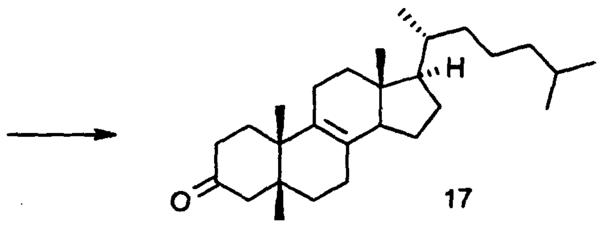

17

17

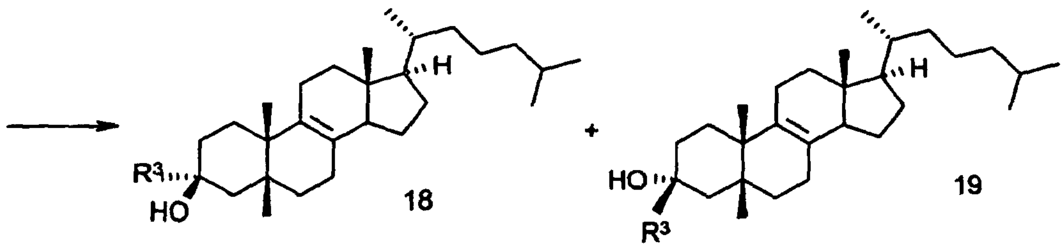

18

+

19

Subsequent reduction of 5β-methylketone **17** is achieved with different well known reducing agents like lithium aluminum hydride, sodium borohydride and diisobutylaluminum hydride [e.g. *Aust.J.Chem.* **35** (1982), 629]. Two diastereomeric alcohols **18** and **19** ($R^3$ = hydrogen) can be obtained and readily separated by column chromatography.

If 5β-methylketone **17** is treated with Grignard reagents, two diastereomeric tertiary alcohols (**18** and **19**; with $R^3$ = $C_1$-$C_6$-alkyl) are obtained, which are easily separated by column chromatography.

Analogues that combine a 5-cyano-substituent with different steroidal sidechains can be synthesized by the following general route (see scheme 6): Starting from lichesterol, which can be synthesized as described in the literature [*J.Chem.Soc.Perkin Trans. 1* (1981), 2125], an enone can be generated by an oppenauer oxidation [e.g. *J.Chem.Soc. Perkin Trans.1* (1994), 2667]. A cyano group can be introduced by conjugate addition [*J.Org.Chem.* **59** (1994), 2766]. After protection of the 3-ketone as an ketal, the side chain can be cleaved by ozonolysis and subsequent reductive work up [*Synthesis* **3** (1990), 193]. The 22-alcohol can be transformed to the corresponding tosylate to generate the appropriate leaving group for the copper catalyzed addition of grignard reagents. By this addition, different alkyl- and aryl side chains can be intoduced [*Chem.Pharm.Bull.* **28** (1980), 606]. After deprotection of the ketone, the latter can be reduced to the corresponding α- and β-alcohols by standard methods.

**Scheme 6:**

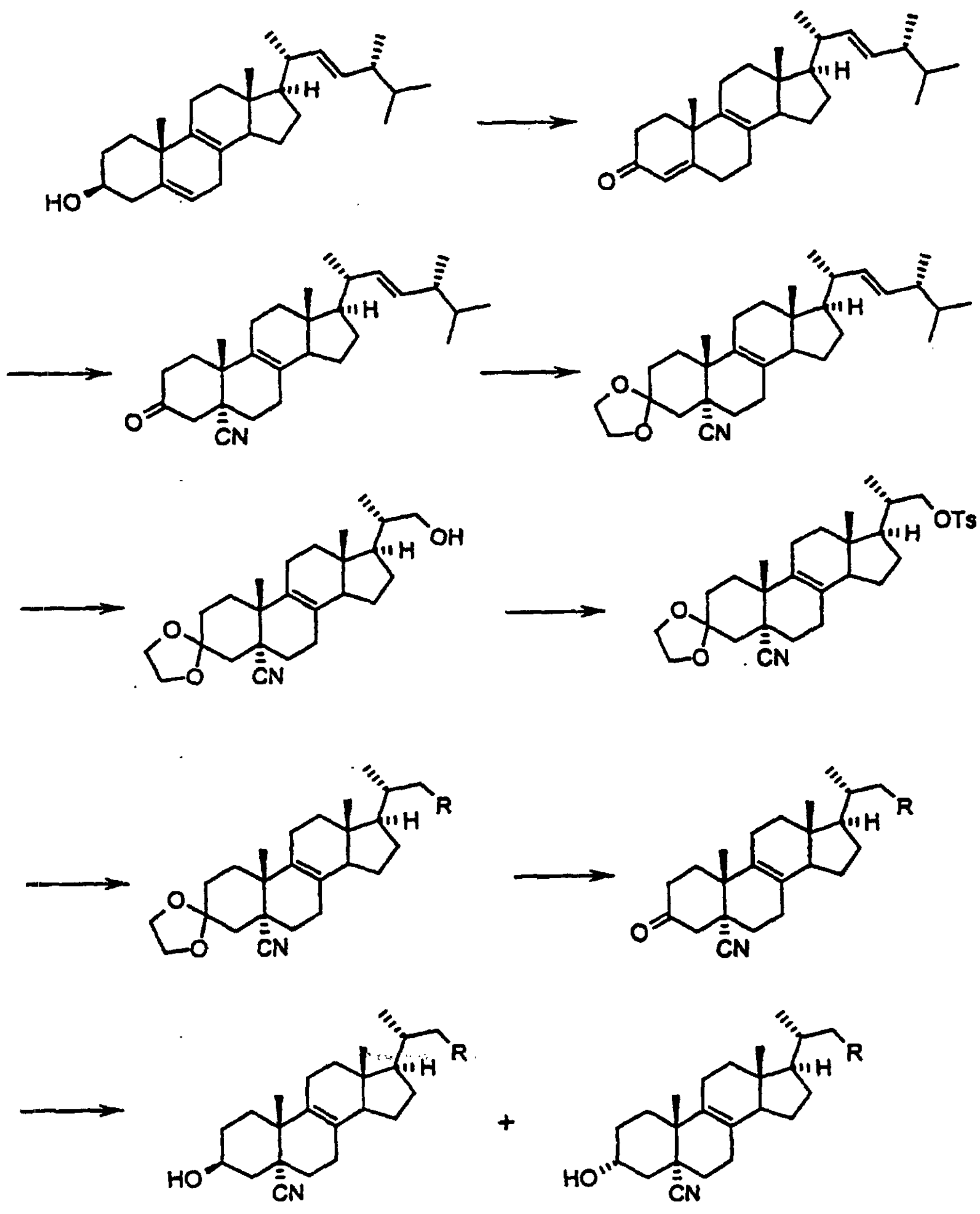

The compounds of the present invention will influence the meiosis in oocytes as well as in male germ cells.

The existence of a meiosis inducing substance in nature has been known for some time. However, until recently the identity of the meiosis inducing substance or substances was unknown.

The prospects of being able to influence the meiosis are several. According to a preferred embodiment of the present invention, a compound of formula I or an ester or salt thereof can be used to stimulate the meiosis. According to another preferred embodiment of the present invention, a compound of formula I or an ester or salt thereof can be

used to stimulate the meiosis in humans. Thus, the compounds of formula I and ester or salts thereof are promising as new fertility regulating agents without the usual side effect on the somatic cells which are known from the hitherto used hormonal contraceptives which are based on estrogens and/or gestagens.

For use as a contraceptive agent in females, a meiosis inducing substance can be administered so as to prematurely induce resumption of meiosis in oocytes while they are still in the growing follicle, before the ovulatory peak of gonadotropins occurs. In women, the resumption of the meiosis can, for example, be induced a week after the preceding menstruation has ceased. When ovulated, the resulting overmature oocytes are then most likely not to be fertilised. The normal menstrual cycle is not likely to be affected. In this connection it is important to notice, that the biosynthesis of progesterone in cultured human granulosa cells (somatic cells of the follicle) is not affected by the presence of a meiosis inducing substance whereas the estrogens and gestagens used in the hitherto used hormonal contraceptives do have an adverse effect on the biosynthesis of progesterone.

According to another aspect of this invention, a meiosis inducing substance of formula I or an ester or salt thereof can be used in the treatment of certain cases of infertility in females, including women, by administration thereof to females who, due to an insufficient own production of meiosis activating substance, are unable to produce mature oocytes. Also, when *in vitro* fertilisation is performed, better results can be achieved, when a compound of formula I or an ester or salt thereof is added to the medium in which the oocytes are cultured.

When infertility in males, including men, is caused by an insufficient own production of the meiosis activating substance and thus a lack of mature sperm cells, administration of a compound of formula I or an ester or salt thereof may relieve the problem.

As an alternative to the method described above, contraception in females can also be achieved by administration of a compound of formula I or an ester or salt thereof which inhibits the meiosis, so that no mature oocytes are produced. Similarly, contraception in males can be achieved by administration of a compound of formula I or an ester or salt thereof which inhibits the meiosis, so that no mature sperm cells are produced.

The route of administration of compositions containing a compound of formula I or an ester or salt thereof may be any route which effectively transports the active compound to its site of action.

Thus, when the compounds of this invention are to be administered to a mammal, they are conveniently provided in the form of a pharmaceutical composition which comprises at least one compound of formula I or an ester or salt thereof in connection with a pharmaceutically acceptable carrier. For oral use, such compositions are preferably in the form of capsules or tablets.

From the above it will be understood that administrative regimen called for will depend on the condition to be treated. Thus, when used in the treatment of infertility, the administration may have to take place once only, or for a limited period, e.g. until pregnancy is achieved. When used as a contraceptive, the compounds of formula I or ester or salts thereof will either have to be administered continuously or cyclically. When used as a contraceptive by females and not taken continuously, the timing of the administration relative to the ovulation will be important.

Pharmaceutical Compositions

Pharmaceutical compositions comprising a compound of formula I or an ester or salt thereof may further comprise carriers, diluents, absorption enhancers, preservatives, buffers, agents for adjusting the osmotic pressure, tablet disintegrating agents and other ingredients which are conventionally used in the art. Examples of solid carriers are magnesium carbonate, magnesium stearate, dextrin, lactose, sugar, talc, gelatin, pectin, tragacanth, methyl cellulose, sodium carboxymethyl cellulose, low melting waxes and cocoa butter.

Liquid compositions include sterile solutions, suspensions and emulsions. Such liquid compositions may be suitable for injection or for use in connection with *ex vivo* and *in vitro* fertilisation. The liquid compositions may contain other ingredients which are conventionally used in the art, some of which are mentioned in the list above.

Further, a composition for transdermal administration of a compound of this invention may be provided in the form of a patch and a composition for nasal administration may be provided in the form of a nasal spray in liquid or powder form.

The dose of a compound of formula I to be used will be determined by a physician and will depend, *inter alia*, on the particular compound employed, on the route of administration and on the purpose of the use. In general, the compositions of the invention are prepared by intimately bringing into association the active compound with the liquid or solid auxiliary ingredients and then, if necessary, shaping the product into the desired formulation.

Usually, not more than 1000 mg, preferably not more than 100 mg, and in some preferred instances not more than 10 mg, of a compound of formula I is to be administered to mammals, e.g. to man, per day.

None of the compounds of formula I have shown to be toxic when administered to man in an amount of 1000 mg per day.

The compounds of formula I thereof can be synthesised by methods known per se.

The present invention is further illustrated by the following examples which, however, are not to be construed as

limiting the scope of protection. The features disclosed in the foregoing description and in the following examples may, in any combination thereof, be material for realising the invention in diverse forms thereof.

EXAMPLE 1

3β-Hydroxy-4,4-dimethyl-5α-chola-8,14-dien-24-oic acid cyclohexyl ester

3β-Hydroxy-4,4-dimethyl-5α-chola-8,14-dien-24-oic acid (0.2 g) is suspended in 10 ml of cyclohexanol, 0.1 ml of boron trifluoride diethyl etherate is added and the mixture is stirred at 55-60°C for 20 hours. After evaporation to dryness under reduced pressure, the remanens is purified by column chromatography and crystallized from methanol to give the title compound (49 mg). Melting point: 130-132°C. $^{1}$H-NMR ($CDCl_3$): δ = 5.35 (1H, s); 4.75 (1H, m); 3.24 (1H, m). MS: Calculated: 482.8. Found 482.4.

EXAMPLE 2

3β-Hydroxy-4,4-dimethyl-24-phenyl-5α-chola-8,14-dien-24-one

3β-*tert*-Butyldimethylsilyloxy-4,4-dimethyl-5α-chola-8,14-dien-24 oic acid-N-methoxy-N-methyl amide (0.57 g) is reacted with phenylmagnesiumbromide and hydrolysed with ethanol/HCl following the procedure outlined in example 6 in WO 99/58549 to give the title compound (0.31 g). Melting point: 196-199°C. $^{1}$H-NMR ($CDCl_3$, 300 MHz): δ = 8.0-7.4 (5H, m); 5.35 (1H, s); 3.24 (1H, m); 3.0 (2H, m). MS: Calculated: 460.7. Found 460.3.

EXAMPLE 3

3β-Hydroxy-4,4-dimethyl-5α-chola-8,14-dien-24-oic acid-N-phenyl amide

3β-*tert*-Butyldimethylsilyloxy-4,4-dimethyl-5α-chola-8,14-dien-24 oic acid (0.50 g) is dissolved in 15 ml of dry dichloromethane. After cooling to -15°C, 0.188 ml of N-methylmorpholine and 0.153 ml of isobutylchloroformiate is added and the mixture is stirred at -15°C for 20 minutes,whereupon 0.44 ml of aniline is added. The mixture is stirred overnight and the temperature is slowly elevated to room temperature. After aqueous work-up and crystallization from methanol, 3β-*tert*-butyldimethylsilyloxy-4,4-dimethyl-5α-chola-8,14-dien-24-oic acid-N-phenyl amide (0.431g) is obtained. H-NMR ($CDCl_3$ ,400 MHz): δ = 7.53 (2H,d); 7.34 (2H,t ); 7.17 (1H,s ); 7.12 (1H,t ); 5.35 (1H,s ); 3.21 (1H,m ); 0.9 (9H,s ); 0.04 (6H,m).

3β-*tert*-Butyldimethylsilyloxy-4,4-dimethyl-5α-chola-8,14-dien-24-oic acid-N-phenyl amide (50 mg) is dissolved in 5 ml of ethanol, 0.2 ml of 6N hydrogen chloride is added and the mixture is stirred at room temperature overnight. After aqueous work-up and crystallization from methanol the title compound is obtained. (36 mg). H-NMR ($CDCl_3$, 400 MHz): δ = 7.53 (2H, d); 7.33 (2H, t); 7.18 (1H, s); 7.12 (1H, t ); 5.36 (1H, s); 3.26 (1H, m). MS: Calculated: 475.7. Found: 475.4.

EXAMPLE 4

4,4-Dimethyl-24-phenylamino-5α-chola-8,14-dien-3β-ol

3β-*tert*-Butyldimethylsilyloxy-4,4-dimethyl-5α-chola-8,14-dien-24 oic acid (0.15 g) is reacted with aniline following the procedure outlined in example 3 and reduced with lithium aluminium hydride (0.15 g) in THF at room temperature. Aqueous work-up and crystallization from methanol gives 3β-*tert*-butyldimethylsilyloxy-4,4-dimethyl-24-phenylamino-5α-chola-8,14-dien (106 mg). $^{1}$H-NMR ($CDCl_3$, 300 MHz)): δ = 7.17 (2H, t); 6.69 (1H, t); 6.6 (2H, d); 5.35 (1H, s); 3.6 (1H, s); 3.2 (1H, m); 3.09 (2H, m); 0.9 (9H, s); 0.04 (6H, m).

3β-*tert*-Butyldimethylsilyloxy-4,4-dimethyl-24-phenylamino-5α-chola-8,14-dien (100 mg) is hydrolysed with ethanol/HCl at 50°C. Aqueous work-up and crystallization from ethanol gives the title compound (53 mg). Melting point: 178-180°C. $^{1}$H-NMR ($CDCl_3$, 300 MHz)): δ = 7.19 (2H, t); 6.7 (1H, t); 6.63 (2H, d); 5.36 (1H, s); 3.6 (1H, s); 3.26 (1H, m); 3.1 (2H, m). MS: Calculated: 461.7. Found: 461.3.

EXAMPLE 5

(20R)-4,4,20-Trimethyl-21-phenyl-5α-pregna-8,14-dien-3β-ol

5A: According to *Steroids* **26** (1975), p. 339-357, the 4,4-dimethylstigmasterole has been synthesised.

5B: Ozonolysis of compound 5A at -70°C by slowly introduction of $O_3$, and following reduction at -70°C by sodium-bis-(2-methoxyethoxy) aluminium hydride and further reduction at a temperature about -30°C with lithium aluminium hydride gave afforded 23-nor-4,4-dimethyl-5α-cholest-5-en-3β,22-diol, which was diacetylated in pyridine with acetic acid anhydride. (1B).

5C: Compound 5B was isomerised by reflux in ethanol/6M hydrochloric acid giving 23-nor-4,4-dimethyl-5α-cholest-8,14-dien-3β,22-diol.

5D: Compound 5C was selectively C-22 tosylated by treatment of *p*-toluene sulfonyl chloride in pyridine by standing over night in room temperature. The compound was purified by colom chromathography and crystalised.

NMR: H ppm : 0.78 s $CH_3$; 0.82 s $CH_3$; 0.98 d $CH_3$; 1.02 s $CH_3$ ; 2.46 s $CH_3$-aromat; 3.22 m H 3 a; 5.38 s H15; 7.33 d 2H; 7,78 d 2H.

This intermediate, 5D, is used in several of the following examples.

5E: Compound 5D was reacted with phenylmagnesium bromide catalysed by $Li_2CuCl_4$ giving the title compound. (*Chem.Pharm.Bull*. **28** (1980), p. 606-611; Masuo Monsaki *et al*.).

NMR: H ppm : 0.82 d $CH_3$; 0.83 s $CH_3$; 1.02 s $CH_3$; 1.03 s $CH_3$; 2.55 m 1H; 2,91 dd 1H; 3.25 m 3 a H; 5.4 s 1H (15); 7.17 m 2H; 7.28 m 3H.

EXAMPLE 6

(20R)-4,4,20-Trimethyl-21-(3-methylphenyl)-5α-pregna-8,14-dien-3β-ol

According to example 5 (compound 5D) was reacted with 3-methylphenyl magnesium bromide and $Li_2CuCl_4$ giving the title compound.

NMR: H ppm: 0.80 d $CH_3$; 0.82 2s 2$CH_3$; 1.0 2s 2$CH_3$; 2.33 s $CH_3$-aromat; 3.27 m H3 a 5.40 s 1H; 6.98 m 3H; 7.17 1H.

EXAMPLE 7

(20R)-4,4,20-Trimethyl-21-(4-methylphenyl)-5α-pregna-8,14-dien-3β-ol

According to example 5 (compound 5D) was reacted with 4-methylphenyl magnesium bromide and $Li_2CuCl_4$ giving the title compound.

NMR: H ppm: 0.84 s, 2d 3 $CH_3$; 1.04 2s 2$CH_3$; 2.33 s $CH_3$-aromat; 3.25 m H3 a; 5.40 s 1H; 7.05 m 4H.

EXAMPLE 8

(20R)-4,4,20-Trimethyl-21-(2-methylphenyl)-5α-pregna-8,14-dien-3β-ol

According to example 5 (compound 5D) was reacted with 2-methylphenyl magnesium bromide and $Li_2CuCl_4$ giving the title compound. NMR: H ppm: 0.84 s + 2d, 3 $CH_3$; 1.04 2s 2$CH_3$ ; 2.32 s $CH_3$-aromat; 3.25 m H3 a ; 5.40 s 1H; 7.10 m 4H;

EXAMPLE 9

(20R)-4,4,20-Trimethyl-21-(cyclohexyl)-5α-pregna-8,14-dien-3β-ol

According to example 5 (compound 5D) was reacted with cyclohexyl magnesium bromide and $Li_2CuCl_4$ giving the title compound.

NMR: H: mmp: 0.81 s $CH_3$ ; 0.82 s $CH_3$; 0.90 d $CH_3$ ; 1.03 s $CH_3$ ; 104 s $CH_3$ ; 3.24 s H3 a; 5.37 s H ;
MS : 424.4.

EXAMPLE 10

(20R)-4,4,20-Trimethyl-22-(phenyl)-5α-pregna-8,14-dien-3β-ol

According to example 5 (compound 5D) was reacted with tolyl magnesium bromide and $Li_2CuCl_4$ giving the

title compound.

NMR: H ; ppm : 0.80 s $CH_3$; 0.82 s $CH_3$; 1.02 s $CH_3$; 1.04 s $CH_3$; 1.06 d $CH_3$; 3.25 m H3 a; 5.35 s H; 7.18 m 3H ; 7.27 m 2H.

EXAMPLE 11

(20R)-4,4,20-Trimethyl-21-(3-hydroxyphenyl)-5α-pregna-8,14-dien-3β-ol

According to example 19 (compound 19 D) was reacted with 3-trimethylsilyloxyphenyl magnesium bromide and $Li_2CuCl_4$ giving the title compound.

NMR: H; ppm : 0.80 d $CH_3$; 0.83 s $CH_3$; 0.84 s $CH_3$; 1.01 s $CH_3$; 1.03 s $CH_3$; 3.26 m H3 a; 5.40 s H; 6.66 m 2H; 6.76 m H; 7.16 m H .

MS : 434.3.

EXAMPLE 12

(20R)-4,4,20-Trimethyl-22-(cyclohexyl)-5α-pregna-8,14-dien-3β-ol

According to example 5 (compound 5D) was reacted with cyclohexylmethyl magnesium bromide and $CuLi_2Cl_4$ giving the title compound.

NMR: H ppm : 0.80 s $CH_3$; 0.83 s $CH_3$; 0.91 d $CH_3$; 1.01 s $CH_3$; 1.03 S $CH_3$; 3.24 m H3 a; 5.35 s H.

MS : 483.3.

EXAMPLE 13

(20R)-4,4,20-Trimethyl-21-(cyclobutyl)-5α-pregna-8,14-dien-3β-ol

In the reaction of example 28 in WO 99/58549 there was further isolated a compound which was identified as the title compound.

NMR : H ; ppm ; 0.80 s $CH_3$; 0.84 s $CH_3$; 0.89 d $CH_3$; 1.01 s $CH_3$; 1.03 s $CH_3$; 3.25 m H3 a; 5.35 s 1H.

EXAMPLE 14

(20R)-4,4,20-Trimethyl-21-(cyclopentyl)-5α-pregna-8,14-dien-3β-ol

According to example 5 (compound 5D) was reacted with cyclopentyl magnesium bromide and $Li_2CuCl_4$ giving the title compound.

NMR : H ; ppm : 0.80 s $CH_3$; 0.82 s $CH_3$; 0.94 d $CH_3$, 1.01 s $CH_3$; 1.03 s $CH_3$; 3.25 m H3 a; 5.35 s H.

EXAMPLE 15

27-Nor-3β-hydroxy-4,4-dimethyl-5α-cholesta-8,14-dien-26-oic acid benzyl ester

The compound is synthesised following the procedures outlined in example 20, below.
$^1$H-NMR ($CDCl_3$, 400 MHz): $\delta$ = 7.35 (5H, m); 5.34 (1H, s); 5.11 (2H, s); 3.23 (1H, m).

EXAMPLE 16

3β-Hydroxy-4,4-dimethyl-5α-chola-8,14-dien-24-oic acid-N-(4-methylpiperazinyl)amide

3β-*tert*-Butyldimethylsilyloxy-4,4-dimethyl-5α-chola-8,14-dien-24-oic acid (0.40 g) is reacted with N-methylpiperazine and hydrolysed with HCl/ethanol following the procedure outlined in example 39 in WO 99/58549 to give

the title compound (80 mg). Melting point: 189-191 °C.

$^1$H-NMR ($CDCl_3$, 300 MHz): δ = 5.35 (1H, s); 3.63 (2H, m); 3.48 (2H, m); 3.24 (1H, m); 2.31 (3H, s). MS: Calculated: 482.8. Found 482.3.

EXAMPLE 17

3β-Hydroxy-4,4-dimethyl-5α-chola-8,14-dien-24-oic acid-N-(isonipecotic acid ethyl ester)amide

3β-*tert*-Butyldimethylsilyloxy-4,4-dimethyl- 5α-chola-8,14-dien-24-oic acid (0.50 g) is reacted with ethylisonipecotate and hydrolysed with HCl/ethanol following the procedure outlined in example 39 in WO 99/58549 to give the title compound (85 mg). Melting point: 116-119°C.

$^1$H-NMR ($CDCl_3$, 400 MHz): δ = 5.35 (1H, s); 4.43 (1H, m); 4.15 (2H, q); 3.82 (1H, m); 3.25 (1H, m); 3.11 (1H, m); 2.8 (1H, m); 1.28 (3H, t). MS: Calculated: 539.8. Found 539.4.

EXAMPLE 18

3β-Hydroxy-4,4-dimethyl-5α-chola-8,14-dien-24-oic acid-N-(phenylalanine methyl ester)amide

3β-*tert*-Butyldimethylsilyloxy-4,4-dimethyl-5α-chola-8,14-dien-24-oic acid (0.40 g) is reacted with phenylalanine methyl ester and hydrolysed with HCl/methanol following the procedures outlined in example 39 in WO 99/58549 to give the title compound (86 mg). Melting point: 158-160°C.

$^1$H-NMR ($CDCl_3$, 400 MHz): δ = 7.27 (3H, m); 7.09 (2H, m); 5.86 (1H, d); 5.35 (1H, s); 4.89 (1H, m); 3.73 (3H, s); 3.25 (1H, m); 3.13 (2H, m). MS: Calculated: 561.8. Found 561.5.

EXAMPLE 19

4,4-Dimethyl-24-benzyloxy-5α-chola-8,14-dien-3β-ol

The compound is synthesised following the procedure in example 50 in WO 99/58549. Melting point: 114-115°C.

$^1$H-NMR ($CDCl_3$, 300 MHz): δ = 7.38-7.23 (5H, m); 5.35 (1H, s); 4.51 (2H, s); 3.45 (2H, m), 3.24 (1H, m). MS: Calculated: 476.7. Found: 476.3.

EXAMPLE 20

3β-Hydroxy-4,4-dimethyl-5α-chola-8,14-dien-24-oic acid benzyl ester

3β-Hydroxy-4,4-dimethyl-5α-chola-8,14-dien-24-oic acid (100 mg) is suspended in 5 ml of dry DMF. 811 mg of cesium carbonate and 0.29 ml of benzyl chloride is added and the mixture is stirred at 50°C overnight. After aqueous work-up, column chromatography and crystallisation from acetone/water 55 mg of the title compound is obtained. Melting point: 118-119°C.

$^1$H-NMR ($CDCl_3$, 300 MHz): δ = 7.35 (5H, m); 5.35 (1H, s); 5.12 (2H, s); 3.23 (1H, m). MS: Calculated: 490.7. Found: 490.3.

EXAMPLES 21 + 22

(20R)-5-Cyano-21-cyclohexyl-20-methyl-5α-pregn-8-en-3β-ol and (20R)-5-cyano-21-cyclohexyl-20-methyl-5α-pregn-8-en-3α-ol

a) Ergosta-4,8,22-trien-3-one

1.90 g ergosta-5,8,22-trien-3β-ol (lichesterol) were treated with 0.50 g aluminum-tris-isopropylate as described in example 56 in WO 99/58549. Column chromatography gave 1.54 g ergosta-4,8,22-trien-3-one as a white solid.

$^1$H-NMR ($CDCl_3$): δ = 0.69 (s, 3H, H-18); 0.82-1.05 (4 x d, 4 x Me); 1.36 (s, 3H, H-19); 5.22 (m, 2H, H-22/23); 5.77 (s, 1H, H-4)

b) 5-Cyano-5α-ergosta-8,22-dien-3-one

1.54 g ergosta-4,8,22-trien-3-one were treated with 11.73 ml diethylaluminum cyanide solution (1N, toluene) as described in example 58 in WO 99/58549. After aqueous work up and chromatography 0.90 g 5-cyano-5α-ergosta-8,22-dien-3-one were isolated as a white solid (beside the corresponding 5β-cyano-compound).

$^1$H-NMR ($CDCl_3$): δ = 0.66 (s, 3H, H-18); 0.82-1.05 (4 x d, 4 x Me); 1.28 (s, 3H, H-19); 5.21 (m, 2H, H-22/23)

c) 5-Cyano-3-(spiro-2',5'-dioxa-cyclopentyl)-5α-ergosta-8,22-diene

A mixture of 900 mg 5-cyano-5α-ergosta-8,22-dien-3-one, 0.97 ml ethylene glycol, 25 mg p-toluenesulfonic acid in 20 ml toluene was refluxed at a dean-stark-trap for 2 hours. After cooling, the reaction mixture was poured into saturated sodium bicarbonate solution, extracted with ethyl acetate and washed with water. The combined organic extracts were dried and evaporated to give 900 mg 5-cyano-3-(spiro-2',5'-dioxa-cyclopentyl)-5α-ergosta-8,22-diene as a white solid.

$^1$H-NMR ($CDCl_3$): δ = 0.63 (s, 3H, H-18); 0.82-1.05 (4 x d, 4 x Me); 1.11 (s, 3H, H-19); 3.90-4.15 (m, 4H, 3-ketal); 5.20 (m, 2H, H-22/23)

d) (20R)-5-Cyano-3-(spiro-2',5'-dioxa-cyclopentyl)-5α-pregn-8-ene-20-methanol

450 mg 5-cyano-3-(spiro-2',5'-dioxa-cyclopentyl)-5α-ergosta-8,22-diene were treated with ozone as described in example 5b. After reductive work up, 172 mg (20R)-5-cyano-3-(spiro-2',5'-dioxa-cyclopentyl)-5α-pregn-8-ene-20-methanol were isolated as a white solid.

$^1$H-NMR ($CDCl_3$): δ = 0.65 (s, 3H, H-18); 1.06 (d, J=7 Hz, 3H, H-21); 1.10 (s, 3H, H-19); 3.35 (m, 1H, H-22); 3.66 (m, 1H, H-22); 3.90-4.12 (m, 4H, 3-ketal)

e) (20R)-5-Cyano-3-(spiro-2',5'-dioxa-cyclopentyl)-5α-pregn-8-ene-20-methanol-4-methylbenzene sulfonate

1.13 g (20R)-5-cyano-5α-pregn-8-ene-20-methanol was treated with 869 mg p-toluenesulfonyl chloride as described in example 5d. After aqueous work up and column chromatography, 1.19 g (20R)-5-cyano-3-(spiro-2',5'-dioxa-cyclopentyl)-5α-pregn-8-ene-20-methanol-4-methylbenzene sulfonate were isolated.

$^1$H-NMR ($CDCl_3$): δ = 0.59 (s, 3H, H-18); 1.00 (d, J=7 Hz, 3H, H-21); 1.10 (s, 3H, H-19); 3.70-4.12 [m, 4H (3-ketal) + 2H (H-22)]

f) (20R)-5-Cyano-21-cyclohexyl-20-methyl-3-(spiro-2',5'-dioxa-cyclopentyl)-5α-pregn-8-ene

258 mg (20R)-5-cyano-3-(spiro-2',5'-dioxa-cyclopentyl)-5α-pregn-8-ene-20-methanol-4-methylbenzene sulfonate were treated with cyclohexyl magnesium bromide analoguously to example 5e. After column chromatography, 147 mg (20R)-5-cyano-21-cyclohexyl-20-methyl-3-(spiro-2',5'-dioxa-cyclopentyl)-5α-pregn-8-ene were isolated as a pale yellow solid.

$^1$H-NMR ($CDCl_3$): δ = 0.62 (s, 3H, H-18); 0.90 (d, J=7 Hz, 3H, H-21); 1.10 (s, 3H, H-19); 3.90-4.12 (m, 4H, 3-ketal)

g) (20R)-5-Cyano-21-cyclohexyl-20-methyl-5α-pregn-8-en-3-one

A mixture of 128 mg (20R)-5-cyano-21-cyclohexyl-20-methyl-3-(spiro-2',5'-dioxacyclopentyl)-5α-pregn-8-ene, 31 mg amberlyste 15 and 8 ml acetone was stirred at room temperature for 20 hours. After filtration and evaporation of the solvent 94 mg (20R)-5-cyano-21-cyclohexyl-20-methyl-5α-pregn-8-en-3-one were isolated.

$^1$H-NMR ($CDCl_3$): δ = 0.67 (s, 3H, H-18); 0.90 (d, J=7 Hz, 3H, H-21); 1.27 (s, 3H, H-19); 2.55 (pseudo-s, 2H)

h) (20R)-5-Cyano-21-cyclohexyl-20-methyl-5α-pregn-8-en-3β-ol and (20R)-5-cyano-21-cyclohexyl-20-methyl-5α-pregn-8-en-3α-ol

90 mg (20R)-5-cyano-21-cyclohexyl-20-methyl-5α-pregn-8-en-3-one were treated with 33 mg sodium borohydride as described in example 58b in WO 99/58549. After column chromatography, 15 mg (20R)-5-cyano-21-cyclohexyl-20-methyl-5α-pregn-8-en-3β-ol and 25 mg (20R)-5-cyano-21-cyclohexyl-20-methyl-5α-pregn-8-en-3α-ol were isolated.

(20R)-5-cyano-21-cyclohexyl-20-methyl-5α-pregn-8-en-3β-ol:
$^1$H-NMR ($CDCl_3$): δ = 0.63 (s, 3H, H-18); 0.93 (d, J=7 Hz, 3H, H-21); 1.11 (s, 3H, H-19); 4.12 (broad-m, 1H, H-3)

(20R)-5-cyano-21-cyclohexyl-20-methyl-5α-pregn-8-en-3α-ol:

$^1$H-NMR ($CDCl_3$): δ = 0.62 (s, 3H, H-18); 0.92 (d, J=7 Hz, 3H, H-21); 1.06 (s, 3H, H-19); 4.12 (narrow-m, 1H, H-3)

EXAMPLES 23 + 24

(20R)-5-Cyano-21-phenyl-20-methyl-5α-pregn-8-en-3β-ol and (20R)-5-cyano-21-phenyl-20-methyl-5α-pregn-8-en-3α-ol

a) (20R)-5-Cyano-21-phenyl-20-methyl-3-(spiro-2',5'-dioxa-cyclopentyl)-5α-pregn-8-ene

400 mg (20R)-5-cyano-3-(spiro-2',5'-dioxa-cyclopentyl)-5α-pregn-8-ene-20-methanol-4-methylbenzene sulfonate (example 21 e) were treated with phenyl magnesium bromide analoguously to example 21f. After column chromatography, 190 mg (20R)-5-cyano-21-phenyl-20-methyl-3-(spiro-2',5'-dioxa-cyclopentyl)-5α-pregn-8-ene were isolated as a white solid.

$^1$H-NMR ($CDCl_3$): δ = 0.65 (s, 3H, H-18); 0.84 (d, J=7 Hz, 3H, H-21); 1.12 (s, 3H, H-19); 2.90 (dd, J=12 hz, J=3 Hz, 1H, H-21); 3.90-4.12 (m, 4H, 3-ketal); 7.12-7.30 (m, 5H, ph)

b) (20R)-5-Cyano-21-phenyl-20-methyl-5α-pregn-8-en-3-one

180 mg (20R)-5-cyano-21-phenyl-20-methyl-3-(spiro-2',5'-dioxa-cyclopentyl)-5α-pregn-8-ene were treated with amberlyste 15 as described in example 21g. After filtration and evaporation of the solvent, 164 mg (20R)-5-cyano-21-phenyl-20-methyl-5α-pregn-8-en-3-one were isolated.

$^1$H-NMR ($CDCl_3$): δ = 0.68 (s, 3H, H-18); 0.84 (d, J=7 Hz, 3H, H-21); 1.27 (s, 3H, H-19); 2.55 (pseudo-s, 2H); 2.90 (dd, J=12 hz, J=3 Hz, 1H, H-21); 7.12-7.30 (m, 5H, ph)

c) (20R)-5-Cyano-21-phenyl-20-methyl-5α-pregn-8-en-3β-ol and (20R)-5-cyano-21-phenyl-20-methyl-5α-pregn-8-en-3α-ol

152 mg (20R)-5-cyano-21-cyclohexyl-20-methyl-5α-pregn-8-en-3-one were treated with 50 mg sodium borohydride as described in example 58b in WO 99/58549. After column chromatography 36 mg (20R)-5-cyano-21-phenyl-20-methyl-5α-pregn-8-en-3β-ol and 46 mg (20R)-5-cyano-21-phenyl-20-methyl-5α-pregn-8-en-3α-ol were isolated.

(20R)-5-Cyano-21-phenyl-20-methyl-5α-pregn-8-en-3β-ol:

$^1$H-NMR ($CDCl_3$): δ = 0.67 (s, 3H, H-18); 0.85 (d, J=7 Hz, 3H, H-21); 1.10 (s, 3H, H-19); 2.90 (dd, J=12 hz, J=3 Hz, 1H, H-21); 4.15 (broad-m, 1H, H-3); 7.12-7.30 (m, 5H, ph)

(20R)-5-Cyano-21-phenyl-20-methyl-5α-pregn-8-en-3α-ol:

$^1$H-NMR ($CDCl_3$): δ = 0.65 (s, 3H, H-18); 0.84 (d, J=7 Hz, 3H, H-21); 1.05 (s, 3H, H-19); 2.90 (dd, J=12 hz, J=3 Hz, 1H, H-21); 4.12 (narrow-m, 1H, H-3); 7.12-7.30 (m, 5H, ph)

EXAMPLE 25

An agonistic oocyte assay can be performed as follows:

Oocytes were obtained from immature female mice (C57BL/6J x DBA/2J F1, Bomholtgaard, Denmark) weighing 13-16 grams, that were kept under controlled temperature (20-22°C), light (lights on 06.00-18.00) and relative humidity (50-70%). The mice received an intra-peritoneal injection of 0.2 ml gonadotropins (Gonal-F, Serono) containing 20 IU FSH and 48 hours later the animals were killed by cervical dislocation.

The ovaries were dissected out and the oocytes were isolated in Hx-medium (see below) under a stereo microscope by manual rupture of the follicles using a pair of 27 gauge needles. Spherical oocytes displaying an intact germinal vesicle (hereinafter designated GV) were divided in cumulus enclosed oocytes (hereinafter designated CEO) and naked oocytes (hereinafter designated NO) and placed in α-minimum essential medium (α-MEM without ribonucleosides, Gibco BRL, Cat. No. 22561 ) supplemented with 3 mg/ml bovine serum albumin (BSA, Sigma Cat. No. A-7030), 5 mg/ml human serum albumin (HSA, Statens Seruminstitute, Denmark), 0.23 mM pyruvate (Sigma, Cat. No S-8636), 2 mM glutamine (Flow Cat. No. 16-801), 100 IU/ml penicillin and 100 μg/ml streptomycin (Flow, Cat No. 16-700). This medium

was supplemented with 3 mM hypoxanthine (Sigma Cat. No. H-9377) and designated Hx-medium.

The oocytes were rinsed three times in Hx-medium and oocytes of uniform size were divided into groups of CEO and NO. CEO and NO were cultured in 4-well multidishes (Nunclon, Denmark) in which each well contained 0.4 ml of Hx-medium. One control well (i.e., 35-45 oocytes cultured in identical medium with no addition of test compound) was always cultured simultaneously with 3 test wells (35-45 oocytes per well supplemented with test compound).

The oocytes were cultured in a humidified atmosphere of 5% $CO_2$ in air for 24 hours at 37°C. By the end of the culture period, the number of oocytes with germinal vesicle (hereinafter designated GV), germinal vesicle breakdown (hereinafter designated GVB) and polar bodies (hereinafter designated PB), respectively, were counted using a stereomicroscope (Wildt, Leica MZ 12). The percentage GVB, defined as percentage of oocytes undergoing GVB per total number of oocytes in that well, was calculated as:

%GVB = (number of GVB + number of PB/ total number oocytes) X 100.

The % PB was defined as percentage of oocytes displaying one extruded polar body per total number of oocytes in that well.

The effect of the tested compounds has been indexed against control level and 4,4-dimethyl-5α-cholesta-8,14,24-trien-3β-ol (hereinafter designated FF-MAS) where controls and FF-MAS are indexed to an effect of 0 and 100, respectively. The relative effect of the tested compound is calculated as follows:

Relative effect = ((test GVB % - control GVB %) / (FF-MAS GVB % - control GVB %)) x 100.

Results

Table 1.

| The mean percentage GVB, the mean percentage PB and mean Relative Effect of compounds after culture of naked oocytes (NO) in vitro for 24 hours. | | | | |
|---|---|---|---|---|
| | Concentration; mikroM | mean % GVB | mean % PB | mean Relative Effect |
| CONTROL | 0 | 11,9 | 5,5 | 0 |
| FF-MAS | 10 | 86,1 | 28,5 | 100 |
| Example 3 | 10 | 10 | 3 | -10 |
| Example 7 | 10 | 82 | 29,5 | 98 |
| Example 9 | 10 | 73,5 | 21,5 | 98 |

EXAMPLE 26

An antagonistic oocyte assay can be performed as follows:

Animals

Oocytes were obtained from immature female mice (C57Bl/6J x DBA/2J F1-hybrids, Bomholtgaard, Denmark) weighing 13-16 grams, that were kept under controlled lighting and temperature. The mice received an intra-peritoneal injection of 0.2 ml gonadotropins (Gonal F, Serono, Solna, Sweden, containing 20 IU FSH, alternatively, Puregon, Organon, Swords, Ireland containing 20 IU FSH) and 48 hours later the animals were killed by cervical dislocation.

Test of meiosis-inhibiting substances in the oocyte test

The ovaries were dissected out and the oocytes were isolated in Hx-medium (see below) under a stereo microscope by manual rupture of the follicles using a pair of 27 gauge needles. Spherical, naked oocytes (NO) displaying an intact germinal vesicle (GV) were placed in α-minimum essential medium (α-MEM without ribonucleosides, Gibco BRL, Cat.No. 22561) supplemented with 3 mM hypoxanthine (Sigma Cat. No. H-9377), 8 mg/ml human serum

albumin (HSA, Statens Seruminstitut, Denmark), 0.23 mM pyrubate (Sigma, Cat. No. S-8636), 2 mM glutamine (Flow Cat. No. 16-801), 100 IU/ml penicillin and 100 μg/ml streptomycin (Flow, Cat No. 16-700). This medium was designated Hx-medium.

Naked oocytes (NO) were rinsed three times in Hx-medium. 4,4-Dimethyl-5α-cholesta-8,14,24-trien-3β-ol (FF-MAS) has previously been shown to induce meiosis in NO in vitro (Byskov, A.G. et al. *Nature* **374** (1995), 559 - 562). NO were cultured in Hx-medium supplemented with 5 μM FF-MAS in co-culture with the test compounds in different concentrations in 4-well multidishes (Nunclon, Denmark) in which each well contained 0.4 ml of the medium and 35-45 oocytes. One positive control (i.e., 35 - 45 oocytes cultured in Hx-medium containing FF-MAS with no addition of test compound) was always run simultaneously with the test cultures, which were supplemented with different concentrations of the compounds to be tested. In addition, one negative control (35 - 45 oocytes cultured in Hx-medium alone) was run simultaneously with the positive control.

Examination of oocytes

By the end of the culture period, the number of oocytes with germinal vesicle (GV) or germinal vesicle breakdown (GVB) and those with polar body (PB) was counted using a stereomicroscope or an inverted microscope with differential interference contrast equipment. The percentage of oocytes with GVB + PB per total number of oocytes were calculated in the test cultures and in the control (positive and negative) culture groups. The relative inhibition of the test compound was calculated by the following formula:

Inhibition of test compound (in percentage) =

$$100 - [(GVB_{\text{test compound}} - GVB_{\text{negative control}}) \times 100/(GVB_{\text{positive control}} - GVB_{\text{negative control}})].$$

In case of a dose response curve, an $IC_{50}$ (dose, which lead to a 50% inhibition) was calculated.

## Claims

1. Use of a compound of the general formula I:

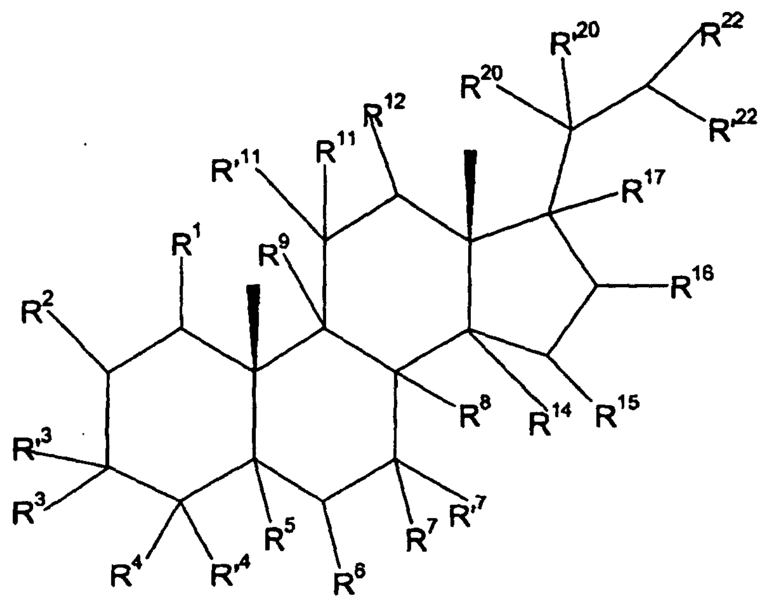

wherein $R^1$ is hydrogen, $R^2$ is hydrogen, $R^3$ is hydrogen or a perfluoro($C_1$-$C_6$ alkyl) group, $R'^3$ is hydroxy; or $R^3$ is, together with $R'^3$, oxo; $R^4$ and $R'^4$, which are different or identical are selected from the group comprising hydrogen and $C_1$-$C_6$ alkyl or $R^4$ designates, together with $R'^4$ and $R^5$, a methano bridge between the carbon atoms in 4 and 5 position or an additional bond between the carbon atoms in 4 and 5 position; $R^5$ is hydrogen, $C_1$-$C_6$

alkyl, cyano, hydroxymethyl, or carbaldehyde, or $R^5$ designates, together with $R^6$, an additional bond between the carbon atoms at which $R^5$ and $R^6$ are placed; $R^6$ is hydrogen; $R^7$ is hydrogen, $R'^7$ is hydrogen; $R^8$ is hydrogen, or $R^8$ designates, together with $R^7$, $R^9$ or $R^{14}$, an additional bond between the carbon atoms at which $R^8$ and $R^7$, $R^9$ or $R^{14}$ are placed; $R^9$ is hydrogen, or $R^9$ designates, together with $R^8$, an additional bond between the carbon atoms at which $R^9$ and $R^8$ are placed; $R^{11}$ is hydrogen, $R'^{11}$ is hydrogen; $R^{12}$ is hydrogen, $R^{14}$ is hydrogen or $R^{14}$ designates, together with $R^{15}$, an additional bond between the carbon atoms at which $R^{14}$ and $R^{15}$ are placed; $R^{15}$ is hydrogen; $R^{16}$ is hydrogen, $R^{17}$ is hydrogen, or $R^{17}$ designates, together with $R^{16}$, an additional bond between the carbon atoms at which $R^{17}$ and $R^{16}$ are placed; $R^{20}$ is $C_1$-$C_6$ alkyl; $R'^{20}$ is hydrogen; $R'^{22}$ is hydrogen; and $R^{22}$ is cyclohexyl; and esters and salts thereof, for the manufacture of a medicament for regulating meiosis.

**2.** The use of (20R)-20-methyl-21-(cyclopentyl)-5α-pregna-8,14-dien-3β-ol; (20R)-20-methyl-21-(cyclohexyl)-5α-pregna-8,14-dien-3β-ol; (20R)-20-methyl-21-(cyclobutyl)-5α-pregna-8,14-dien-3β-ol; (20R)-20-methyl-21-(cyclopentyl)-5α-pregna-5,7-dien-3β-ol; (20R)-20-methyl-21-(cyclohexyl)-5α-pregna-5,7-dien-3β-ol; (20R)-20-methyl-21-(cyclobutyl)-5α-pregna-5,7-dien-3β-ol; (20R)-21-cyclohexyl-5,20-dimethyl-5β-pregn-8(14)-en-3-one; 4,4-dimethyl-24-benzoylamido-5α-chola-8,14-dien-3β-ol; (20R)-4,4,20-trimethyl-21-(cyclohexyl)-5α-pregna-8,14-dien-3β-ol; (20R)-4,4,20-trimethyl-22-(cyclohexyl)-5α-pregna-8,14-dien-3β-ol; (20R)-4,4,20-trimethyl-21-(cyclopentyl)-5α-pregna-8,14-dien-3β-ol; (20R)-20-methyl-21-phenyl-5α-pregna-8,14-dien-3β-ol; (20R)-20-methyl-21-(3-methylphenyl)-5α-pregna-8,14-dien-3β-ol; (20R)-20-methyl-21-(3-hydroxyphenyl)-5α-pregna-8,14-dien-3β-ol; (20R)-20-methyl-21-phenyl-5α-pregna-5,7-dien-3β-ol; (20R)-20-methyl-21-(3-hydroxyphenyl)-5α-pregna-5,7-dien-3β-ol; (20R)-20-methyl-21-phenyl-5α-pregna-5,7-dien-3β-ol; (20R)-5,20-dimethyl-21-phenyl-5β-pregna-8(14)-en-3-one; 4,4-dimethyl-24-benzoylamido-5α-chola-8,14-dien-3β-ol; 4,4-dimethyl-24-benzyloxy-5α-chola-8,14-dien-3β-ol; 3β-hydroxy-4,4-dimethyl-5α-chola-8,14-dien-24-oic acid cyclohexyl ester; 3β-hydroxy-4,4-dimethyl-24-phenyl-5α-chola-8,14-dien-24-one; 3β-hydroxy-4,4-dimethyl-5α-chola-8,14-dien-24-oic acid-N-phenyl amide; 4,4-dimethyl-24-phenylamino-5α-chola-8,14-dien-3β-ol; (20R)-4,4,20-trimethyl-21-phenyl-5α-pregna-8,14-dien-3β-ol; (20R)-4,4,20-trimethyl-21-(3-methylphenyl)-5α-pregna-8,14-dien-3β-ol; (20R)-4,4,20-trimethyl-21-(4-methylphenyl)-5α-pregna-8,14-dien-3β-ol; (20R)-4,4,20-trimethyl-21-(2-methylphenyl)-5α-pregna-8,14-dien-3β-ol; (20R)-4,4,20-trimethyl-22-(phenyl)-5α-pregna-8,14-dien-3β-ol; (20R)-4,4,20-trimethyl-21-(3-hydroxyphenyl)-5α-pregna-8,14-dien-3β-ol; (20R)-4,4,20-trimethyl-21-(cyclobutyl)-5α-pregna-8,14-dien-3β-ol; 27-nor-3β-hydroxy-4,4-dimethyl-5α-cholesta-8,14-dien-26-oic acid benzyl ester; 3β-hydroxy-4,4-dimethyl-5α-chola-8,14-dien-24-oic acid-N-(4-methylpiperazinyl)amide; 3β-hydroxy-4,4-dimethyl-5α-chola-8,14-dien-24-oic acid-N-(isonipecotic acid ethyl ester)amide; 3β-hydroxy-4,4-dimethyl-5α-chola-8,14-dien-24-oic acid-N-(phenylalanine methyl ester)amide; 3β-hydroxy-4,4-dimethyl-5α-chola-8,14-dien-24-oic acid benzyl ester; (20R)-5-cyano-21-cyclohexyl-20-methyl-5α-pregn-8-en-3β-ol; (20R)-5-cyano-21-cyclohexyl-20-methyl-5α-pregn-8-en-3α-ol; (20R)-5-cyano-21-phenyl-20-methyl-5α-pregn-8-en-3β-ol; or (20R)-5-cyano-21-phenyl-20-methyl-5α-pregn-8-en-3α-ol; for the manufacture of a medicament for regulating meiosis.

**3.** Use of a compound according to claim 1 or 2 for the preparation of a medicament for the treatment of infertility in mammals, preferably in humans (males and females).

**4.** Use of a compound according to claim 1 or 2 for the preparation of a contraceptive agent, preferably to humans (males and females).

**5.** Use of a compound according to claim 1 or 2 in a fertilisation culture medium also containing a mammalian germ cell, preferably a human cell.

**6.** Use according to any one of the previous use claims wherein the compound is any of the compounds stated in Claim 2.

**7.** A method of regulating the meiosis in a mammalian germ cell which method comprises extracorporal administering an effective amount of a compound according to claim 1 or 2 to a germ cell in need of such a treatment.

**8.** A method wherein a compound according to claim 1 or 2 is administered to a germ cell by extracorporal administering it to a mammal hosting said cell.

**9.** A method according to any one of the preceding method claims wherein the germ cell the meiosis of which is to be regulated is an oocyte.

**10.** A method according to any one of the previous method claims wherein a compound according to claim 1 or 2 is

administered to an oocyte *ex vivo* or *in vitro*.

**11.** A method according to any one of the previous method claims wherein the germ cell the meiosis of which is to be regulated is a male germ cell.

**12.** A method according to any one of the previous method claims whereby mature male germ cells are produced by administering a compound of the general formula I described above to testicular tissue *ex vivo* or *in vitro*.

**13.** Method according to any one of the previous method claims wherein the compound is any of the compounds stated in Claim 2.

**Patentansprüche**

**1.** Verwendung einer Verbindung nach allgemeiner Formel I:

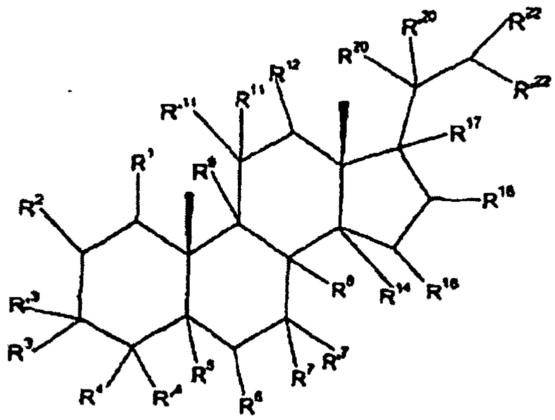

wobei $R^1$ Wasserstoff ist, $R^2$ Wasserstoff ist, $R^3$ Wasserstoff oder eine Perfluor($C_1$-$C_6$ Alkyl) Gruppe ist, $R^{-3}$ Hydroxy ist; oder $R^3$, zusammen mit $R^{-3}$, Oxo ist; $R^4$ und $R^{-4}$, welche verschieden oder identisch sind, werden aus der Gruppe umfassend Wasserstoff und $C_1$-$C_6$ Alkyl ausgewählt, oder $R^4$ bezeichnet, zusammen mit $R^{-4}$ und $R^6$, eine Methanbrücke zwischen den Kohlenstoffatomen an Position 4 und 5 oder eine zusätzliche Bindung zwischen den Kohlenstoffatomen an Position 4 und 5; $R^5$ ist Wasserstoff, $C_1$-$C_8$ Alkyl, Cyano, Hydroxymethyl oder Carbaldehyd, oder $R^5$ bezeichnet, zusammen mit $R^6$, eine zusätzliche Bindung zwischen den Kohlenstoffatomen, an denen $R^5$ und $R^6$ platziert sind; $R^6$ Wasserstoff ist; $R^7$ Wasserstoff ist, $R^{-7}$ Wasserstoff ist; $R^8$ Wasserstoff ist, oder $R^8$ bezeichnet, zusammen mit $R^7$, $R^9$ oder $R^{14}$, eine zusätzliche Bindung zwischen den Kohlenstoffatomen, an denen $R^8$ und $R^7$, $R^9$ oder $R^{14}$ platziert sind; $R^9$ Wasserstoff ist, oder $R^9$ bezeichnet, zusammen mit $R^8$, eine zusätzliche Bindung zwischen den Kohlenstoffatomen, an denen $R^9$ und $R^8$ platziert sind; $R^{11}$ Wasserstoff ist, $R^{-11}$ Wasserstoff ist; $R^{12}$ Wasserstoff ist, $R^{14}$ Wasserstoff ist oder $R^{14}$ bezeichnet, zusammen mit $R^{15}$, eine zusätzliche Bindung zwischen den Kohlenstoffatomen, an denen $R^{14}$ und $R^{15}$ platziert sind; $R^{15}$ Wasserstoff ist; $R^{16}$ Wasserstoff ist, $R^{17}$ Wasserstoff ist, oder $R^{17}$ bezeichnet, zusammen mit $R^{16}$, eine zusätzliche Bindung zwischen den Kohlenstoffatomen, an denen $R^{17}$ und $R^{18}$ platziert sind; $R^{20}$ $C_1$-$C_6$ Alkyl ist; $R^{-20}$ Wasserstoff ist; $R^{-22}$ Wasserstoff ist; und $R^{22}$ Cyclohexyl ist; und Ester und Salze davon, zur Herstellung eines Arzneimittels zur Regulierung der Meiose.

**2.** Die Verwendung von (20R)-20-Methyl-21-(cyclopentyl)-5α-pregna-8,14-dien-3β-ol; (20R)-20-Methyl-21-(cyclohexyl)-5α-pregna-8,14-dien-3β-ol; (20R)-20-Methyl-21-(cyclobutyl)-5α-pregna-8,14-dien-3β-ol; (20R)-20-Methyl-21-(cyclopentyl)-5α-pregna-5,7-dien-3β-ol; (20R)-20-Methyl-21-(cyclohexyl)-5α-pregna-5,7-dien-3β-ol; (20R)-20-Methyl-21-(cyclobutyl)-5α-pregna-5,7-dien-3β-ol; (20R)-21-cyclohexyl-5,20-dimethyl-5β-pregna-8(14)-en-3-on; 4,4-Dimethyl-24-benzoylamido-5α-chola-8,14-dien-3β-ol; (20R)-4,4,20-Trimethyl-21-(cyclohexyl)-5α-pregna-8,14-dien-3β-ol; (20R)-4,4,20-Trimethyl-22-(cyclohexyl) )-5α-pregna-8,14-dien-3β-ol; (20R)-20-Methyl-21-phenyl-5α-pregna-8,14-dien-3β-ol; (20R)-20-Methyl-21-(3-methylphenyl)-5α-pregna-8,14-dien-3β-ol; (20R)-20-Methyl-21-(3-hydroxyphenyl)-5α-pregna-8,14-dien-3β-ol; (20R)20-Methyl-21-phenyl-5α-pregna-5,7-dien-3β-ol; (20R)-20-Methyl-21-phenyl-5α-pregna-5,7-dien-3β-ol; (20R)-5,20-Dimethyl-21-phenyl-5β-pregna-8(14)-en-3-on; 4,4-Dimethyl-24-benzoylamido-5α-chola-8,14-dien-3β-ol; 4,4-Dimethyl-24-benzyloxy-5α-chola-8,14-dien-3β-ol; 3β-Hydroxy-4,4-dimethyl-5α-chola-8,14-dien-24-carbonsäure cyclohexyl ester; 3β-Hydroxy-4,4-dimethyl-24-phenyl-5α-chola-8,14-dien-24-on; 3β-Hydroxy-4,4-dimethyl-24-phenyl-5α-chola-8,14-dien-24-carbonsäure-N-Phenyl Amid; 4,4-Dimethyl-24-phenylamino-5α-chola-8,14-dien-3β-ol; (20R)-4,4,20-Trimethyl-21-phenyl-5α-pregna-8,14-dien-3β-ol; (20R)-4,4,20-Trimethyl-21-(3-methylphenyl)-5α-pregna-8,14-dien-3β-ol; (20R)-4,4,20-Trimethyl-21-(4-methylphenyl)-5α-pregna-8,14-dien-3β-ol; (20R)-4,4,20-Trimethyl-21-(2-methylphenyl)-5α-pregna-8,14-dien-3β-ol; (20R)-4,4,20-Trimethyl-22-(phenyl)-5α-pregna-8,14-dien-3β-ol; (20R)-4,4,20-Trimethyl-21-(3-hydroxylphenyl)-5α-pregna-8,14-dien-3β-ol; (20R)-4,4,20-Trimethyl-21-(cyclobutyl)-5α-pregna-8,14-dien-3β-ol; 27-Nor-3β-hydroxy-4,4-dimethyl-5α-cholesta-8,14-dien-26-carbonsäure benzyl ester; 3β-Hydroxy-4,4-dimethyl-5α-chola-8,14-dien-24-carbonsäure-N-(4-methylpiperazinyl)amid; 3β-Hydroxy-4,4-dimethyl-5α-chola-8,14-dien-24-oicsäure-N-(isonipeconsäureethylester)amid; 3β-Hydroxy-4,4-dimethyl-5α-chola-8,14-dien-24-carbonsäure-N-(phenylalaninethylester)amid; 3β-Hydroxy-4,4-dimethyl-5α-chola-8,14-dien-24-carbonsäure-benzylester; (20R)-5-Cyano-21-cyclohexyl-20-methyl-5α-pregn-8-en-3β-ol; (20R)-5-Cyano-21-cyclohexyl-20-methyl-5α-pregn-8-en-3α-ol; (20R)-5-Cyano-21-phenyl-20-methyl-5α-pregn-8-en-3β-ol; oder (20R)-5-Cyano-21-phenyl-20-methyl-5α-pregn-8-en-3α-ol; zur Herstellung eines Arzneimittels zur Regulierung der Meiose.

**3.** Verwendung einer Verbindung gemäß Anspruch 1 oder 2 zur Herstellung eines Arzneimittels für die Behandlung von Unfruchtbarkeit in Säugetieren, vorzugsweise in Menschen (Männer und Frauen).

**4.** Verwendung einer Verbindung gemäß Anspruch 1 oder 2 zur Herstellung eines Verhütungsmittels, vorzugsweise in Menschen (Männer und Frauen).

**5.** Verwendung einer Verbindung gemäß Anspruch 1 oder 2 in einem Fertilisationskulturmedium, dass auch eine Säugetierkeimzelle enthält, vorzugsweise eine menschliche Zelle.

**6.** Verwendung nach irgendeinen der vorhergehenden Verwendungsansprüche, wobei die Verbindung irgendeine der in Anspruch 2 angegebenen Verbindungen ist.

**7.** Verfahren zur Regulierung der Meiose in einer Säugetierkeimzelle, welches Verfahren die extrakorporale Verabreichung einer wirksamen Menge einer Verbindung gemäß des Anspruches 1 oder 2 einer Keimzelle, die solch eine Behandlung benötigt, umfasst.

**8.** Verfahren, wobei eine Verbindung nach Anspruch 1 oder 2 einer Keimzelle durch extrakorporales Verabreichen in eine Säugetierwirtszelle verabreicht wird.

**9.** Verfahren nach irgendeinem der vorangehenden Verfahrensansprüche, wobei die Keimzelle, deren Meiose reguliert werden muss, eine Oocyte ist.

**10.** Verfahren nach irgendeinem der vorhergehenden Verfahrensansprüche, wobei eine Verbindung gemäß Anspruch 1 oder 2 einer Oocyte *ex vivo* oder *in vitro* verabreicht wird.

**11.** Verfahren nach irgendeinem der vorhergehenden Verfahrensansprüche, wobei die Keimzelle, deren Meiose reguliert werden muss, eine männliche Keimzelle ist.

**12.** Verfahren nach irgendeinem der vorhergehenden Verfahrensansprüche, wobei reife männliche Keimzellen durch *ex vivo* oder *in vitro* Verabreichen einer Verbindung der allgemeinen Formel I, wie oben beschrieben, in ein testikuläres Gewebe produziert werden.

**13.** Verfahren nach irgendeinem der vorhergehenden Verfahrensansprüche, wobei die Verbindung irgendeine der in Anspruch 2 angegebenen Verbindungen ist.

**Revendications**

**1.** Utilisation d'un composé de formule générale I :

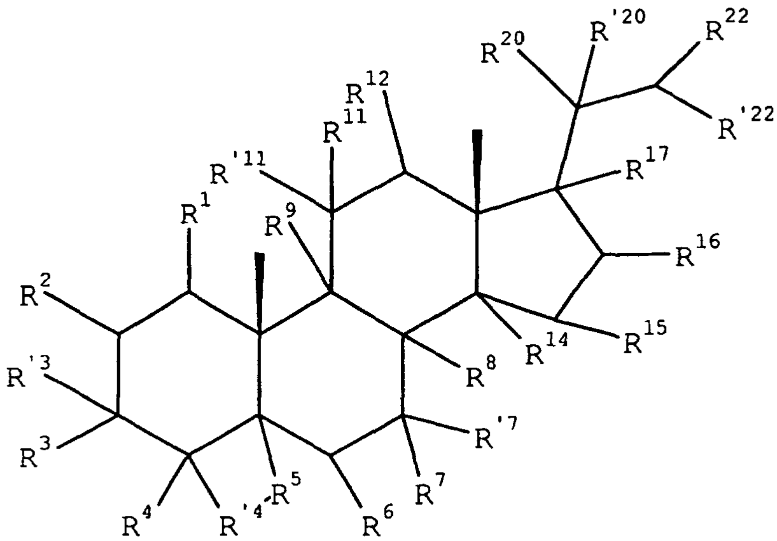

dans laquelle $R^1$ est un atome d'hydrogène, $R^2$ est un atome d'hydrogène, $R^3$ est un atome d'hydrogène ou un groupe perfluoro(alkyle en $C_1$-$C_6$), $R'^3$ est le groupe hydroxy ; ou encore $R^3$, avec $R'^3$, est le groupe oxo ; $R^4$ et $R'^4$, qui sont identiques ou différents, sont choisis dans l'ensemble comprenant l'atome d'hydrogène et les groupes alkyle en $C_1$-$C_6$, ou encore $R^6$, avec $R'^4$ et $R^5$, désignent un pont méthano entre les atomes de carbone sur les positions 4 et 5, ou une liaison additionnelle entre les atomes de carbone sur les positions 4 et 5 ; $R^5$ est un atome d'hydrogène, un groupe alkyle en $C_1$-$C_6$, cyano, hydroxy-méthyle ou carbaldéhyde, ou encore $R^5$, avec $R^6$, désigne une liaison additionnelle entre les atomes de carbone au niveau desquels sont placés $R^5$ et $R^6$ ; $R^6$ est un atome d'hydrogène ; $R^7$ est un atome d'hydrogène ; $R'^7$ est un atome d'hydrogène ; $R^8$ est un atome d'hydrogène, ou $R^8$, avec $R^7$, $R^9$ ou $R^{14}$, désigne une liaison additionnelle entre les atomes de carbone au niveau desquels sont placés $R^5$ et $R^7$, $R^9$ ou $R^{14}$ ; $R^9$ est un atome d'hydrogène, ou encore $R^9$, avec $R^8$, désigne une liaison additionnelle entre les atomes de carbone au niveau desquels sont placés $R^9$ et $R^8$ ; $R^{11}$ est un atome d'hydrogène ; $R'^{11}$ est un atome d'hydrogène ; $R^{12}$ est un atome d'hydrogène, $R^{14}$ est un atome d'hydrogène, ou encore $R^{14}$, avec $R^{15}$, désigne une liaison additionnelle entre les atomes de carbone au niveau desquels sont placés $R^{14}$ et $R^{15}$ ; $R^{15}$ est un atome d'hydrogène ; $R^{16}$ est un atome d'hydrogène, $R^{17}$ est un atome d'hydrogène, ou encore $R^{17}$, avec $R^{16}$, désigne une liaison additionnelle entre les atomes de carbone au niveau desquels sont placés $R^{17}$ et $R^{16}$ ; $R^{20}$ est un groupe alkyle en $C_1$-$C_6$ ; $R'^{20}$ est un atome d'hydrogène ; $R'^{22}$ est un atome d'hydrogène ; et $R^{22}$ est le groupe cyclohexyle ; et de ses esters et sels, pour préparer un médicament destiné à la régulation de la méiose.

**2.** Utilisation, pour préparer un médicament destiné à la régulation de la méiose, du (20R)-20-méthyl-21-(cyclopentyl)-5α-prégna-8,14-diène-3β-ol ; du (20R)-20-méthyl-21-(cyclohexyl)-5α-prégna-8,14-diène-3β-ol ; du (20R)-20-méthyl-21-(cyclobutyl)-5α-prégna-8,14-diène-3β-ol ;du (20R)-20-méthyl-21-(cyclopentyl)-5α-prégna-5,7-diène-3β-ol ; du (20R)-20-méthyl-21-(cyclohexyl)-5α-pregna-5,7-diène-3β-ol ; du (20R)-20-méthyl-21-(cyclobutyl)-5α-pregna-5,7-diène-3β-ol ; de la (20R)-21-cyclohexyl-5,20-diméthyl-5β-pregn-8(14)-ène-3-one ; du 4,4-diméthyl-24-benzoylamido-5α-chola-8,14-diène-3β-ol ; du (20R)-4,4,20-triméthyl-21-(cyclohexyl)-5α-pregna-8,14-diène-3β-ol ; du (20R)-4,4,20-triméthyl-22-(cyclohexyl)-5α-pregna-8,14-diène-3β-ol ; du (20R)-4,4,20-triméthyl-21-(cyclopentyl)-5α-pregna-8,14-diène-3β-ol ; du (20R)-20-méthyl-21-phényl-5α-pregna-8,14-diène-3β-ol ; du (20R)-20-méthyl-21-(3-méthylphényl)-5α-pregna-8,14-diène-3β-ol ; du (20R)-20-méthyl-21-(3-hydroxyphényl)-5α-pregna-8,14-diène-3β-ol ; du (20R)-20-méthyl-21-phényl-5α-pregna-5,7-diène-3β-ol ; du (20R)-20-méthyl-21-(3-hydroxyphényl)-5α-pregna-5,7-diène-3β-ol ; du (20R)-20-méthyl-21-phényl-5α-pregna-5,7-diène-3β-ol ; de la (20R)-5,20-diméthyl-21-phényl-5β-pregna-8(14)-ène-3-one ; du 4,4-diméthyl-24-benzoylamido-5α-chola-8,14-diène-3β-ol ; du 4,4-diméthyl-24-benzyloxy-5α-chola-8,14-diène-3β-ol ; de l'ester cyclohexylique de l'acide 3β-hydroxy-4,4-diméthyl-5α-chola-8,14-diène-24-oïque ; de la 3β-hydroxy-4,4-diméthyl-24-phényl-5α-chola-8,14-diène-24-one ; du N-phénylamide de l'acide 3β-hydroxy-4,4-diméthyl-5α-chola-8,14-diène-24-oïque ; du 4,4-diméthyl-24-phénylamino-5α-chola-8,14-diène-3β-ol ; du (20R)-4,4,20-triméthyl-21-phényl-5α-pregna-8,14-diène-3β-ol ; du (20R)-4,4,20-triméthyl-21-(3-méthylphényl)-5α-pregna-8,14-diène-3β-ol ; du (20R)-4,4,20-triméthyl-21-(4-méthylphényl)-5α-pregna-8,14-diène-3β-ol ; du (20R)-4,4,20-triméthyl-21-(2-méthylphényl)-5α-pregna-8,14-diène-3β-ol ; du (20R)-4,4,20-triméthyl-22-(phényl)-5α-pregna-8,14-diène-3β-ol ; du (20R)-4,4,20-triméthyl-21-(3-hydroxyphényl)-5α-pregna-8,14-diène-3β-ol ; du (20R)-4,4,20-triméthyl-21-(cyclobutyl)-5α-pregna-8,14-diène-3β-ol ; de l'ester benzylique de l'acide 27-nor-3β-hydroxy-4,4-diméthyl-5α-cholesta-8,14-diène-26-oïque ; du N-(4-méthylpipérazinyl)amide de l'acide 3β-hydroxy-4,4-diméthyl-5α-chola-8,14-diène-24-oïque ; du N-(ester éthylique de l'acide isonipécotique)-amide de l'acide 3β-hydroxy-4,4-diméthyl-5α-chola-8,14-diène-24-oïque ; du N-(ester méthylique de la phénylalanine)amide de l'acide 3β-hydroxy-4,4-diméthyl-5α-chola-8,14-diène-24-oïque ; de l'ester benzylique de l'acide 3β-hydroxy-4,4-diméthyl-5α-chola-8,14-diène-24-oïque ; du (20R)-5-cyano-21-cyclohexyl-20-méthyl-5α-pregn-8-ène-3β-ol ; du (20R)-5-cyano-21-cyclohexyl-20-méthyl-5α-pregn-8-ène-3α-ol ; du (20R)-5-cyano-21-phényl-20-méthyl-5α-pregn-8-ène-3β-ol ; ou du (20R)-5-cyano-21-phényl-20-méthyl-5α-pregn-8-ène-3α-ol.

**3.** Utilisation d'un composé selon la revendication 1 ou 2 pour préparer un médicament destiné au traitement de la stérilité chez des mammifères, de préférence chez l'homme (mâles et femelles).

**4.** Utilisation d'un composé selon la revendication 1 ou 2 pour préparer un agent contraceptif, de préférence chez l'homme (de sexe masculin et féminin).

**5.** Utilisation d'un composé selon la revendication 1 ou 2 dans un milieu de culture de fertilisation contenant aussi une cellule germinale de mammifère, de préférence une cellule humaine.

**6.** Utilisation selon l'une quelconque des revendications d'utilisation précédentes, pour laquelle le composé est l'un quelconque des composés mentionnés dans la revendication 2.

**7.** Procédé pour réguler la méiose dans une cellule germinale de mammifère, lequel procédé comprend l'administration extracorporelle d'une quantité efficace d'un composé selon la revendication 1 ou 2 à une cellule germinale ayant besoin d'un tel traitement.

**8.** Procédé dans lequel un composé selon la revendication 1 ou 2 est administré à une cellule germinale par son administration extracorporelle à un mammifère dans lequel se trouve ladite cellule.

**9.** Procédé selon l'une quelconque des revendications de procédé précédentes, dans lequel la cellule germinale dont il s'agit de réguler la méiose est un ovocyte.

**10.** Procédé selon l'une quelconque des revendications de procédé précédentes, dans lequel un composé selon la revendication 1 ou 2 est administré à un ovocyte ex vivo ou in vitro.

**11.** Procédé selon l'une quelconque des revendications de procédé précédentes, dans lequel la cellule germinale dont il s'agit de réguler la méiose est une cellule germinale mâle.

**12.** Procédé selon l'une quelconque des revendications de procédé précédentes, dans lequel les cellules germinales mâles à maturité sont produites par administration d'un composé de formule générale I décrit ci-dessus à un tissu testiculaire ex vivo ou in vitro.

**13.** Procédé selon l'une quelconque des revendications de procédé précédentes, dans lequel le composé est l'un quelconque des composés mentionnés dans la revendication 2.